# EUROPEAN PATENT APPLICATION

(11) **EP 3 476 859 A1**
(43) Date of publication of application: **01.05.2019**
(21) Application number: 17815499.3
(22) Date of filing: 23.06.2017
(51) Int. Cl.: C07K 14/435, C07K 19/00, C12N 1/15, C12N 1/19, C12N 1/21, C12N 5/10, C12N 15/09

(54) **MODIFIED FIBROIN**

(30) Priority: 23.06.2016 JP 2016124674
(71) Applicant: Spiber Inc., Yamagata 997-0052 (JP); Kojima Industries Corporation, Toyota-shi, Aichi 471-8588 (JP)
(72) Inventor: MORITA, Keisuke, Tsuruoka-shi Yamagata 997-0052 (JP); KOTAKA, Koichi, Tsuruoka-shi Yamagata 997-0052 (JP)
(74) Representative: Handley, Matthew Edward
(86) International application number: PCT/JP2017/023116
(87) International publication number: WO 2017/222034

(57) **Abstract**

The present invention relates to a modified fibroin including a domain sequence represented by Formula 1: [(A)ₙ motif-REP]ₘ, in which the domain sequence has an amino acid sequence locally containing a region with locally high hydropathy index equivalent to an amino acid sequence in which one or a plurality of amino acid residues in REP are substituted with amino acid residues with a high hydropathy index and/or one or a plurality of amino acid residues with a high hydropathy index are inserted into REP, as compared to naturally occurring fibroin. In Formula 1, (A)ₙ motif represents an amino acid sequence consisting of 4 to 20 amino acid residues and the number of alanine residues relative to the total number of amino acid residues in the (A)ₙ motif is 83% or more. REP represents an amino acid sequence consisting of 10 to 200 amino acid residues. m represents an integer of 10 to 300, A plurality of (A)ₙ motifs and the like may be the same amino acid sequence or different amino acid sequences.

## Description

### Technical Field

The present invention relates to a modified fibroin. More particularly, the present invention relates to a modified fibroin having an amino acid sequence with a locally high hydropathy index. The present invention also relates to a nucleic acid encoding a modified fibroin, an expression vector including the nucleic acid sequence, a host transformed with the expression vector, and a product made from a modified fibroin.

### Background Art

Fibroin is a type of fibrous protein and contains up to 90% of glycine, alanine and serine residues leading to the formation of a β-pleated sheet (Non-Patent Literature 1). Proteins (silk proteins, hornet silk proteins, and spider silk proteins) and the like constituting the yarn produced by insects and spiders are known as fibroin.

Silk proteins have excellent mechanical properties, hygroscopic properties and deodorizing properties and are widely used as raw materials for garments. In addition, the silk yarn is an immuno-tolerant natural fiber and has high biocompatibility and is therefore also used for surgical sutures.

Up to seven types of silk glands exist in spider, each producing fibroin (spider silk protein) with different properties. According to the organ of the source, spider silk proteins are designated a major ampullate spider protein (MaSp) with high toughness, a minor ampullate spider protein (MiSp) with high elongation, and flagelliform (Flag), tubuliform, aggregate, aciniform, and pyriform spider silk proteins. In particular, structural studies have been intensively conducted in the major ampullate spider protein exhibiting high toughness due to having excellent strength and elongation (Patent Literature 1 and Patent Literature 2).

As a structure specific to fibroin, a structure in which amino acid motifs classified as GPGXX, an extended region rich in alanine residues ((A)ₙ or (GA)ₙ), GGX, and a spacer are repeated is known (Non-Patent Literature 2). In addition, it has been reported that substitution of the (GA)ₙ motif with the (A)ₙ motif leads to decreased elongation but increased tensile strength, an increasing number of GPGXX motifs leads to increased elongation, and substitution of several GPGXX motifs with the (A)ₙ motifs leads to increased tensile strength (Patent Literature 2). In addition, the GGX and GPGXX motifs are thought to have a flexible helical structure that imparts elasticity to yarns (Patent Literature 3).

Recombinant spider silk proteins and recombinant silk proteins are produced in several heterologous protein production systems. For example, transgenic goat, transgenic silkworm, or recombinant plant or mammalian cells are utilized (Non-Patent Literature 3). However, these production systems exhibit a low production rate and are not suitable for mass production meeting the commercial level (Patent Literature 4 and Patent Literature 5). Although many cases of production of recombinant fibroin by organisms such as yeast, mold, gram-negative bacterium and gram-positive bacterium as a production system capable of mass production have also been reported and certain outcomes have been achieved, it has not been possible to achieve industrial mass production of the recombinant fibroin having excellent elongation and tensile strength (Patent Literature 5).

Molded articles obtained by molding spider yarn proteins into various shapes are known. For example, Patent Literature 6 discloses a film using a spider yarn protein. Also, Patent Literature 7 discloses polypeptide particles derived from spider yarn protein.

### Citation List

### Patent Literature

[Patent Literature 1] Japanese Unexamined Patent Publication No. 2012-55269
[Patent Literature 2] Japanese Unexamined Patent Publication No. 2005-502347
[Patent Literature 3] Japanese Unexamined Patent Publication No. 2009-505668
[Patent Literature 4] Japanese Unexamined Patent Publication No. 2014-502140
[Patent Literature 5] International Patent Publication No. WO2015/042164
[Patent Literature 6] Japanese Unexamined Patent Publication No. 2008-507260
[Patent Literature 7] International Patent Publication No. WO2014/61043

### Non Patent Literature

[Non-Patent Literature 1] Asakura et al., Encyclopedia of Agricultural Science, Academic Press: New York, NY, 1994, Vol. 4, pp. 1-11
[Non-Patent Literature 2] Microbial Cell Factories, 2004, 3:14
[Non-Patent Literature 3] Science, 2002, Vol. 295, pp. 472-476

### Summary of Invention

### Problems to be Solved by the Invention

Due to its excellent properties, fibroin has drawn attention as a new material in various industrial fields such as medicine, aviation, and clothing. Also, due to its applicability to molded articles such as a film, which has been produced from a material derived from petroleum conventionally, fibroin has drawn attention as an alternative material (bioplastic). However, it is necessary to further improve the productivity of fibroin in order to achieve a quantity required for commercial level production.

An object of the present invention is to provide a modified fibroin having improved productivity and having improved bending strength when formed into a molded article.

### Means for Solving the Problems

As a result of extensive studies on methods capable of industrial mass production, the present inventors have found that when modified to a fibroin having an amino acid sequence with a locally high hydropathy index, it is possible to improve productivity and improve the bending strength of a molded article produced with the fibroin. The present invention has been completed on the basis of such novel findings.

That is, the present invention relates to, for example, each of the following inventions.
[1] A modified fibroin, including:
   a domain sequence represented by Formula 1: [(A)ₙ motif-REP]ₘ,
   in which the domain sequence has an amino acid sequence containing a region with locally high hydropathy index equivalent to an amino acid sequence in which one or a plurality of amino acid residues in REP are substituted with amino acid residues with a high hydropathy index and/or one or a plurality of amino acid residues with a high hydropathy index are inserted into REP, as compared to naturally occurring fibroin.
   [In Formula 1, (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, a plurality of REPs may be the same amino acid sequence or different amino acid sequences.]
[2] The modified fibroin according to [1], in which the region with locally high hydropathy index is constituted by two to four consecutive amino acid residues.
[3] The modified fibroin according to [1] or [2], in which the amino acid residues with a high hydropathy index are selected from isoleucine (I), valine (V), leucine (L), phenylalanine (F), cysteine (C), methionine (M), and alanine (A).
[4] A modified fibroin, including:
   a domain sequence represented by Formula 1: [(A)ₙ motif-REP]ₘ,
   in which in a case where in all REPs included in a sequence excluding a sequence from a (A)ₙ motif located the most C-terminal side to a C-terminal of the domain sequence from the domain sequence, the total number of amino acid residues contained in a region where an average value of hydropathy indices of four consecutive amino acid residues is 2.6 or more is set as x, and the total number of amino acid residues contained in the sequence excluding the sequence from the (A)ₙ motif located the most C-terminal side to the C-terminal of the domain sequence from the domain sequence is set as y, x/y is 6.2% or more,.
   [In Formula 1, the (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, and a plurality of REPs may be the same amino acid sequence or different amino acid sequences.]
[5] The modified fibroin according to any one of [1] to [4], in which the fibroin has, in addition to the amino acid sequence in which one or a plurality of amino acid residues in REP are substituted with amino acid residues with a high hydropathy index and/or one or a plurality of amino acid residues with a high hydropathy index are inserted into REP, an amino acid sequence corresponding to substitution, deletion, insertion and/or addition of one or a plurality of amino acid residues, as compared to naturally occurring fibroin.
[6] The modified fibroin according to [5], in which the naturally occurring fibroin is a fibroin derived from an insect or a spider.
[7] The modified fibroin according to [5], in which the naturally occurring fibroin is a major ampullate spider protein (MaSp) or minor ampullate spider protein (MiSp) of spiders.
[8] A modified fibroin, including:
   an amino acid sequence set forth in SEQ ID NO: 2, SEQ ID NO: 4 or SEQ ID NO: 5, or
   an amino acid sequence having 90% or more sequence identity with the amino acid sequence set forth in SEQ ID NO: 2, SEQ ID NO: 4 or SEQ ID NO: 5.
[9] The modified fibroin according to any one of [1] to [8], further including a tag sequence at either or both of a N-terminal and a C-terminal.
[10] The modified fibroin according to [9], in which the tag sequence includes an amino acid sequence set forth in SEQ ID NO: 6.
[11] A modified fibroin, including:
   an amino acid sequence set forth in SEQ ID NO: 8, SEQ ID NO: 10 or SEQ ID NO: 11, or
   an amino acid sequence having 90% or more sequence identity with the amino acid sequence set forth in SEQ ID NO: 8, SEQ ID NO: 10 or SEQ ID NO: 11.
[12] A nucleic acid that encodes the modified fibroin according to [1] to [11].
[13] A nucleic acid that hybridizes with a complementary strand of the nucleic acid according to [12] under stringent conditions and encodes a modified fibroin including a domain sequence represented by Formula 1: [(A)ₙ motif-REP]ₘ.
   [In Formula 1, the (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, and a plurality of REPs may be the same amino acid sequence or different amino acid sequences.]
[14] A nucleic acid having 90% or more sequence identity with the nucleic acid according to [13] and encoding a modified fibroin including a domain sequence represented by Formula 1: [(A)ₙ motif-REP]ₘ.
   [In Formula 1, the (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, and a plurality of REPs may be the same amino acid sequence or different amino acid sequences.]
[15] An expression vector, comprising:
   the nucleic acid sequence according to any one of [12] to [14]; and
   one or a plurality of regulatory sequences operably linked thereto.
[16] The expression vector according to [15], which is a plasmid vector or a viral vector.
[17] A host transformed with the expression vector according to [15] or [16].
[18] The host according to [17], which is a prokaryote.
[19] The host according to [18], wherein the prokaryote is a microorganism belonging to a genus selected from the group consisting of *Escherichia, Brevibacillus, Serratia, Bacillus*, *Microbacterium, Brevibacterium, Corynebacterium* and *Pseudomonas.*
[20] The host according to [17], which is a eukaryote.
[21] The host according to [20], in which the eukaryote is a yeast, a filamentous fungus or an insect cell.
[22] The host according to [21], in which the yeast is a yeast belonging to a genus selected from the group consisting of *Saccharomyces, Schizosaccharomyces, Kluyveromyces, Trichosporon, Schwanniomyces, Pichia, Candida, Yarrowia* and *Hansenula.*
[23] The host according to [22], in which the yeast belonging to the genus *Saccharomyces* is *Saccharomyces cerevisiae,* the yeast belonging to the genus *Schizosaccharomyces* is *Schizosaccharomyces pombe,* and the yeast belonging to the genus *Kluyveromyces* is *Kluyveromyces lactis*, the yeast belonging to the genus *Trichosporon* is *Trichosporon pullulans*, the yeast belonging to the genus *Schwaniomyces* is *Schwanniomyces alluvius*, the yeast belonging to the genus *Pichia* is *Pichia pastoris,* the yeast belonging to the genus *Candida* is *Candida albicans*, the yeast belonging to the genus *Yarrowia* is *Yarrowia lipolytica*, and the yeast belonging to the genus *Hansenula* is *Hansenula polymorpha.*
[24] The host according to [21], in which the filamentous fungus is a filamentous fungus belonging to a genus selected from the group consisting of *Aspergillus*, *Penicillium* and *Mucor.*
[25] The host according to [24], in which the filamentous fungus belonging to the genus *Aspergillus* is *Aspergillus oryzae,* the filamentous fungus belonging to the genus *Penicillium* is *Penicillium chrysogenum,* and the filamentous fungus belonging to the genus *Mucor* is *Mucor fragilis.*
[26] The host according to [21], in which the insect cell is a lepidopteran insect cell.
[27] The host according to [26], in which the insect cell is an insect cell derived from *Spodoptera frugiperda* or an insect cell derived from *Trichoplusia ni.*
[28] A product including the modified fibroin according to any one of [1] to [11] and selected from the group consisting of a fiber, a yarn, a filament, a film, a foam, a sphere, a nanofibril, a hydrogel, a resin and an equivalent thereof.

### Effects of the Invention

According to the present invention, it is possible to provide a modified fibroin having improved productivity and having improved bending strength when formed into a molded article.

### Brief Description of Drawings

FIG. 1 is a schematic diagram showing a domain sequence of a modified fibroin.
FIG. 2 is a diagram showing a distribution of values of x/y (%) of naturally occurring fibroin.
FIG. 3 is a schematic cross-sectional diagram showing an embodiment of a press molding apparatus used for manufacturing a molded article.
FIG. 4 is a process diagram schematically showing an embodiment of a method for manufacturing a molded article.

### Embodiments for Carrying Out the Invention

Hereinafter, embodiments for carrying out the present invention will be described in detail. However, the present invention is not limited to the following embodiments.

### [Modified fibroin]

The modified fibroin according to the present invention is a protein including a domain sequence represented by Formula 1: [(A)ₙ motif-REP]ₘ. In the modified fibroin, an amino acid sequence (N-terminal sequence and C-terminal sequence) may be further added to either or both of the N-terminal side and the C-terminal side of the domain sequence. The N-terminal sequence and the C-terminal sequence, although not limited thereto, are typically regions that do not have repetitions of amino acid motifs characteristic of fibroin and consist of amino acids of about 100 residues.

The term "modified fibroin" as used herein means a fibroin whose domain sequence is different from the amino acid sequence of naturally occurring fibroin. The term "naturally occurring fibroin" as used herein is also a protein including a domain sequence represented by Formula 1: [(A)ₙ motif-REP]ₘ.

The "modified fibroin" may be a fibroin whose amino acid sequence has been modified based on naturally occurring fibroin (for example, a fibroin whose amino acid sequence has been modified by altering a gene sequence of cloned naturally occurring fibroin) or a fibroin artificially designed and synthesized independently of naturally occurring fibroin (for example, a fibroin having a desired amino acid sequence by chemically synthesizing a nucleic acid encoding the designed amino acid sequence), as long as it has the amino acid sequence specified in the present invention.

The term "domain sequence" as used herein refers to an amino acid sequence which produces a crystalline region (typically, corresponds to (A)ₙ motif of an amino acid sequence) and an amorphous region (typically, corresponds to REP of an amino acid sequence) peculiar to fibroin and means an amino acid sequence represented by Formula 1: [(A)ₙ motif-REP]ₘ. Here, the (A)ₙ motif represents an amino acid sequence consisting of 4 to 20 amino acid residues and the number of alanine residues relative to the total number of amino acid residues in the (A)ₙ motif is 83% or more. The REP represents an amino acid sequence consisting of 10 to 200 amino acid residues. m represents an integer of 10 to 300. A plurality of (A)ₙ motifs may be the same amino acid sequence or different amino acid sequences. A plurality of REPs may be the same amino acid sequence or different amino acid sequences.

The (A)ₙ motif may be such that the number of alanine residues is 83% or more relative to the total number of amino acid residues in the (A)ₙ motif, preferably 86% or more, more preferably 90% or more, still more preferably 95% or more, and even still more preferably 100% (which means that the (A)ₙ motif consists of only alanine residues). It is preferred that at least seven of a plurality of (A)ₙ motifs in the domain sequence consist of only alanine residues. The phrase "consist of only alanine residues" means that the (A)ₙ motif has an amino acid sequence represented by (A)ₙ (where A represents an alanine residue and n represents an integer of 4 to 20 and preferably an integer of 4 to 16). It is preferable that at least one motif selected from GGX and GPGXX (here, X represents an amino acid residue other than a glycine residue) is contained in the amino acid sequence of REP. With REP containing these motifs, the elongation of fibroin is allowed to be improved.

A modified fibroin according to an embodiment of the present invention may include a domain sequence having an amino acid sequence locally containing a region with a high hydropathy index equivalent to an amino acid sequence in which one or a plurality of amino acid residues in REP are substituted with amino acid residues with a high hydropathy index and/or one or a plurality of amino acid residues with a high hydropathy index are inserted into REP, as compared to naturally occurring fibroin.

It is preferable that in the modified fibroin according to the present embodiment, the region with locally high hydropathy index is constituted by two to four consecutive amino acid residues. With this configuration, the effect of the present invention is more significantly exhibited.

It is more preferable that in the modified fibroin according to the present embodiment, the amino acid residues with a high hydropathy index are selected from isoleucine (I), valine (V), leucine (L), phenylalanine (F), cysteine (C), methionine (M), and alanine (A). With this configuration, the effect of the present invention of improvement of productivity of the fibroin in the recombinant protein production system and remarkable improvement of bending strength of the molded article produced with the fibroin can be more stably exerted.

The modified fibroin according to the present embodiment may further have modifications of an amino acid sequence corresponding to substitution, deletion, insertion and/or addition of one or a plurality of amino acid residues as compared to naturally occurring fibroin, in addition to the amino acid sequence in which one or a plurality of amino acid residues in REP are substituted with amino acid residues with a high hydropathy index and/or one or a plurality of amino acid residues with a high hydropathy index are inserted into REP, as compared to naturally occurring fibroin.

The modified fibroin according to the present embodiment may be obtained by, with respect to a gene sequence of cloned naturally occurring fibroin, substituting one or a plurality of hydrophilic amino acid residues in REP (for example, amino acid residues having a negative hydropathy index) with a hydropathy amino acid residues (for example, amino acid residues having a positive hydropathy index), and/or inserting one or a plurality of hydropathy amino acid residues into REP. Further, for example, the modified fibroin according to the present embodiment may also be obtained by designing an amino acid sequence equivalent to an amino acid sequence in which with respect to the amino acid sequence of naturally occurring fibroin, one or a plurality of hydrophilic amino acid residues in REP are substituted with hydropathy amino acid residues and/or one or a plurality of hydropathy amino acid residues are inserted into REP, and chemically synthesizing a nucleic acid encoding the designed amino acid sequence. In any case, with respect to the amino acid sequence naturally occurring fibroin, in addition to the modification corresponding to substitution of one or a plurality of hydrophilic amino acid residues in REP with hydropathy amino acid residues and/or insertion of one or a plurality of hydropathy amino acid residues into REP, further modification of amino acid sequence corresponding to substitution, deletion, insertion and/or addition of one or a plurality of amino acid residues may be carried out. The substitution, deletion, insertion and/or addition of amino acid residues may be carried out by methods well known to those skilled in the art, such as site-directed mutagenesis. Specifically, the modifications may be carried out by a method described in literature such as Nucleic Acid Res. 10, 6487 (1982), and Methods in Enzymology, 100, 448 (1983).

Naturally occurring fibroin is a protein including a domain sequence represented by Formula 1: [(A)ₙ motif-REP]ₘ, specifically, for example, a fibroin produced by insects or spiders.

Examples of the fibroin produced by insects include silk proteins produced by silkworms such as *Bombyx mori, Bombyx mandarina*, *Antheraea yamamai*, *Anteraea pernyi*, *Eriogyna pyretorum*, *Pilosamia Cynthia ricini*, *Samia cynthia*, *Caligura japonica*, *Antheraea mylitta*, and *Antheraea assama*; and hornet silk proteins discharged by larvae of *Vespa simillima xanthoptera.*

A more specific example of the fibroin produced by insects may be a silkworm fibroin L chain (GenBank Accession No. M76430 (nucleotide sequence), AAA27840.1 (amino acid sequence)).

Examples of the fibroin produced by spiders include spider silk proteins produced by spiders belonging to the genus *Araneus* such as *Araneus ventricosus, Araneus diadematus, Araneus pinguis, Araneus pentagrammicus* and *Araneus nojimai,* spiders belonging to the genus *Neoscona* such as *Neoscona scylla*, *Neoscona nautica*, *Neoscona adianta* and *Neoscona scylloides,* spiders belonging to the genus *Pronus* such as *Pronous minutes,* spiders belonging to the genus *Cyrtarachne* such as *Cyrtarachne bufo* and *Cyrtarachne inaequalis,* spiders belonging to the genus *Gasteracantha* such as *Gasteracantha kuhli* and *Gasteracantha mammosa*, spiders belonging to the genus *Ordgarius* such as *Ordgarius hobsoni* and *Ordgarius sexspinosus*, spiders belonging to the genus *Argiope* such as *Argiope amoena*, *Argiope minuta* and *Argiope bruennich,* spiders belonging to the genus *Arachnura* such as *Arachnura logio,* spiders belonging to the genus *Acusilas* such as *Acusilas coccineus,* spiders belonging to the genus *Cytophora* such as *Cyrtophora moluccensis*, *Cyrtophora exanthematica* and *Cyrtophora unicolor*, spiders belonging to the genus *Poltys* such as *Poltys illepidus*, spiders belonging to the genus *Cyclosa* such as *Cyclosa octotuberculata*, *Cyclosa sedeculata*, *Cyclosa vallata* and *Cyclosa atrata*, and spiders belonging to the genus *Chorizopes* such as *Chorizopes nipponicus*; and spider silk proteins produced by spiders belonging to the genus *Tetragnatha* such as *Tetragnatha praedonia*, *Tetragnatha maxillosa*, *Tetragnatha extensa* and *Tetragnatha squamata*, spiders belonging to the genus *Leucauge* such as *Leucauge magnifica*, *Leucauge blanda* and *Leucauge subblanda*, spiders belonging to the genus *Nephila* such as *Nephila clavata* and *Nephila pilipes,* spiders belonging to the genus *Menosira* such as *Menosira ornata*, spiders belonging to the genus *Dyschiriognatha* such as *Dyschiriognatha tenera*, spiders belonging to the genus *Latrodectus* such as *Latrodectus mactans*, *Latrodectus hasseltii*, *Latrodectus geometricus* and *Latrodectus tredecimguttatus*, and spiders belonging to the family *Tetragnathidae* such as spiders belonging to the genus *Euprosthenops.* Examples of spider silk proteins include traction fiber proteins such as MaSp (MaSp1 and MaSp2) and ADF (ADF3 and ADF4), and MiSp (MiSp1 and MiSp2).

More specific examples of the fibroin produced by spiders include fibroin-3 (adf-3) [derived from *Araneus diadematus]* (GenBank Accession Number AAC47010 (amino acid sequence), U47855 (nucleotide sequence)), fibroin-4 (adf-4) [derived from *Araneus diadematus*] (GenBank Accession Number AAC47011 (amino acid sequence), U47856 (nucleotide sequence)), dragline silk protein spidroin 1 [derived from *Nephila clavipes*] (GenBank Accession Number AAC04504 (amino acid sequence), U37520 (nucleotide sequence)), major angullate spidroin 1 [derived from *Latrodectus hesperus*] (GenBank Accession Number ABR68856 (amino acid sequence), EF595246 (nucleotide sequence)), dragline silk protein spidroin 2 [derived from *Nephila clavata*] (GenBank Accession Number AAL32472 (amino acid sequence), AF441245 (nucleotide sequence)), major anpullate spidroin 1 [derived from *Euprosthenops australis*] (GenBank Accession Number CAJ00428 (amino acid sequence), AJ973155 (nucleotide sequence)) and major ampullate spidroin 2 [*Euprosthenops australis*] (GenBank Accession Number CAM32249.1 (amino acid sequence), AM490169 (nucleotide sequence)), minor ampullate silk protein 1 [*Nephila clavipes*] (GenBank Accession Number AAC14589.1 (amino acid sequence), minor ampullate silk protein 2 [*Nephila clavipes*] (GenBank Accession Number AAC14591.1 (amino acid sequence)), and minor ampullate spidroin-like protein [*Nephilengys cruentata*] (GenBank Accession Number ABR37278.1 (amino acid sequence)).

As a further specified example of naturally occurring fibroin, fibroin whose sequence information is registered in NCBI GenBank may be mentioned. For example, sequences thereof may be confirmed by extracting sequences in which spidroin, ampullate, fibroin, "silk and polypeptide", or "silk and protein" is described as a keyword in DEFINITION among sequences containing INV as DIVISION among sequence information registered in NCBI GenBank, sequences in which a specific character string of products is described from CDS, or sequences in which a specific character string is described from SOURCE to TISSUE TYPE.

A modified fibroin according to another embodiment includes a domain sequence represented by Formula 1: [(A)ₙ motif-REP]ₘ and has an amino acid sequence in which x/y is 6.2% or more, in a case where in all REPs included in a sequence excluding a sequence from a (A)ₙ motif located to most C-terminal side to the C-terminal of the domain sequence from the domain sequence, the total number of amino acid residues contained in a region where an average value of hydropathy indices of four consecutive amino acid residues is 2.6 or more is set as x, and the total number of amino acid residues contained in the sequence excluding the sequence from the (A)ₙ motif located the most C-terminal side to the C-terminal of the domain sequence from the domain sequence is set as y.

Regarding the hydropathy index of amino acid residues, known indices from (Hydropathy index: Kyte J, & Doolittle R (1982) "A simple method for displaying the hydropathic character of a protein", J. Mol. Biol., 157, pp. 105-132) may be used as a reference. Specifically, the hydropathy index (hereinafter also referred to as "HI") of each amino acid is as shown in Table 1 below.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The calculation method of x/y will be described in more detail. In the calculation, the sequence excluding the sequence from the (A)ₙ motif located at the most C-terminal side to the C-terminal of the domain sequence from the domain sequence (hereinafter also referred to as "sequence A") is used. First, in all REPs included in the sequence A, average values of hydropathy indices of four consecutive amino acid residues are calculated. The average value of the hydropathy indices is obtained by dividing the sum total of HI of each of the amino acid residues contained in the four consecutive amino acid residues by 4 (number of amino acid residues). The average value of the hydropathy indices is obtained for all of the four consecutive amino acid residues (each of the amino acid residues is used for calculating the average value 1 to 4 times). Next, a region where the average value of the hydropathy indices of the four consecutive amino acid residues is 2.6 or more is specified. Even if a certain amino acid residue is determined to be included in the "four consecutive amino acid residues having an average value of the hydropathy indices of 2.6 or more" multiple times, the amino acid residue is counted as one amino acid residue in the region. The total number of amino acid residues included in the region is set as x. The total number of amino acid residues included in the sequence A is set as y.

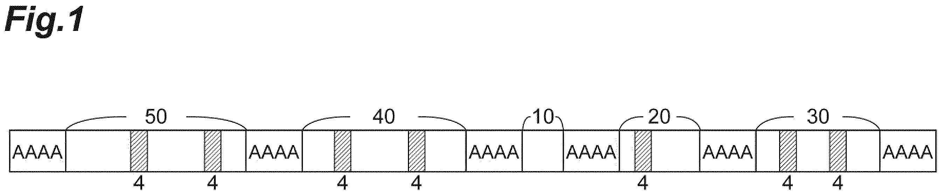

For example, in a case where the feature "four consecutive amino acid residues whose average value of the hydropathy indices is 2.6 or more" is extracted from 20 places (no overlap), in the region where the average value of the hydropathy indices of four consecutive amino acid residues is 2.6 or more, the number of the four consecutive amino acid residues (no overlap) is 20, and thus x is 20 × 4 = 80. In addition, for example, when two of the "four consecutive amino acid residues having an average value of the hydropathy indices of 2.6 or more" overlap by one amino acid residue, in the region where the average value of the hydropathy indices of the four consecutive amino acid residues is 2.6 or more, the number of amino acid residues being included is 7(x = 2 × 4 - 1=7. "-1" is the deduction of overlap). For example, in the case of the domain sequence shown in FIG. 1, since there are seven four consecutive amino acid residues having an average value of the hydropathy indices of 2.6 or more, x is 7 × 4 = 28. In addition, for example, in the case of the domain sequence shown in FIG. 1, y is 4 + 50 + 4 + 40 + 4 + 10 + 4 + 20 + 4 + 30 = 170 (excluding the last (A)ₙ motif located on the most C-terminal side). Next, x/y (%) can be calculated by dividing x by y. In the case of FIG. 1, x/y is 28/170 = 16.47%.

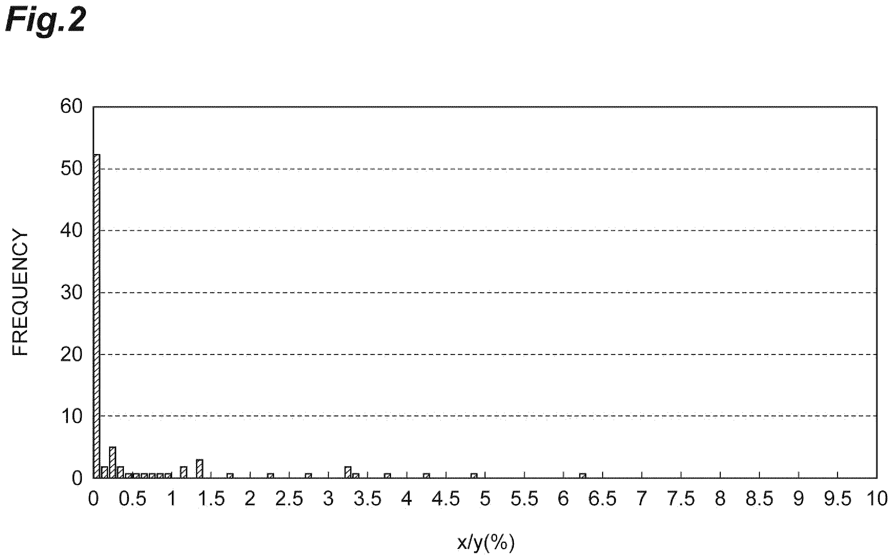

Here, x/y in naturally occurring fibroin will be described. First, as described above, 663 types of fibroins (415 types of fibroins derived from spiders among them) were extracted by confirming fibroins with amino acid sequence information registered in NCBI GenBank by the method exemplified. Among all the extracted fibroins, x/y was calculated by the above-mentioned calculation method with respect to the naturally occurring fibroins including the domain sequence represented by Formula 1: [(A)ₙ motif-REP]ₘ. The results are shown in FIG. 2. In FIG. 2, the horizontal axis represents x/y (%) and the vertical axis represents frequency. As is clear from FIG. 2, x/y in naturally occurring fibroin is less than 6.2% (highest, 6.12%).

In the modified fibroin according to the present embodiment, x/y is preferably 6.2% or more, more preferably 7% or more, still more preferably 10% or more, even still more preferably 20% or more, and still further preferably 30% or more. The upper limit of x/y is not particularly limited, but it may be 45% or less, for example.

The modified fibroin according to the present embodiment may be obtained by, for example, modifying an amino acid sequence of cloned naturally occurring fibroin into the amino acid sequence locally containing a region with locally high hydropathy index by substituting one or a plurality of hydrophilic amino acid residues in REP (for example, amino acid residues having a negative hydropathy index) with hydropathy amino acid residues (for example, amino acid residues having a positive hydropathy index), and/or inserting one or a plurality of hydropathy amino acid residues into REP, such that the x/y condition is satisfied. Alternatively, the modified fibroin according to the present embodiment may also be obtained, for example, by designing an amino acid sequence satisfying the x/y condition based on the amino acid sequence of naturally occurring fibroin and chemically synthesizing a nucleic acid encoding the designed amino acid sequence. In any case, in addition to the modification corresponding to substitution of one or a plurality of amino acid residues in REP with amino acid residues with a high hydropathy index and/or insertion of one or a plurality of amino acid residues with a high hydropathy index into REP, as compared to the amino acid sequence of naturally occurring fibroin, further modification corresponding to substitution, deletion, insertion, and/or addition of one or a plurality of amino acid residues may be carried out.

The amino acid residues with a high hydropathy index is preferably isoleucine (I), valine (V), leucine (L), phenylalanine (F), cysteine (C), methionine (M), and alanine (A), and more preferably valine (V), leucine (L), and isoleucine (I), but not particularly thereto.

A more specific example of the modified fibroin according to the present invention may be a modified fibroin containing (i) an amino acid sequence set forth in SEQ ID NO: 2, SEQ ID NO: 4 or SEQ ID NO: 5, or (ii) an amino acid sequence having 90% or more sequence identity with the amino acid sequence set forth in SEQ ID NO: 2, SEQ ID NO: 4 or SEQ ID NO: 5.

The modified fibroin of (i) will be described. The amino acid sequence set forth in SEQ ID NO: 1 is obtained by deleting consecutive alanine residues in the (A)ₙ motif of an amino acid sequence in naturally occurring fibroin such that the number of the consecutive alanine residues becomes five. The amino acid sequence set forth in SEQ ID NO: 2 is obtained by inserting an amino acid sequences consisting of three amino acid residues (VLI) at two sites for each REP with respect to the amino acid sequence set forth in SEQ ID NO: 1, and deleting a part of the amino acid on the N-terminal side therefrom such that the molecular weight thereof is set to be approximately the same as that of the amino acid sequence set forth in SEQ ID NO: 1. The amino acid sequence set forth in SEQ ID NO: 3 is obtained by inserting two alanine residues at the C-terminal side of each (A)ₙ motif of the amino acid sequence set forth in SEQ ID NO: 1, and further substituting a part of glutamine (Q) residues with serine (S) residues and deleting a part of amino acids on the N-terminal side therefrom such that the molecular weight thereof becomes approximately the same as that of SEQ ID NO: 1. The amino acid sequence set forth in SEQ ID NO: 4 is obtained by inserting an amino acid sequence consisting of three amino acid residues (VLI) at one site for each REP with respect to the amino acid sequence set forth in SEQ ID NO: 3. The amino acid sequence set forth in SEQ ID NO: 5 is obtained by inserting an amino acid sequences consisting of three amino acid residues (VLI) at two sites for each REP with respect to the amino acid sequence set forth in SEQ ID NO: 3.

The modified fibroin of (i) may consist of the amino acid sequence set forth in SEQ ID NO: 2, SEQ ID NO: 4 or SEQ ID NO: 5.

The modified fibroin of (ii) includes an amino acid sequence having 90% or more sequence identity with the amino acid sequence set forth in SEQ ID NO: 2, SEQ ID NO: 4 or SEQ ID NO: 5. The modified fibroin of (ii) is also a protein including a domain sequence represented by Formula 1: [(A)ₙ motif-REP]ₘ. The sequence identity is preferably 95% or more.

The modified fibroin of (ii) preferably has 90% or more sequence identity with the amino acid sequence set forth in SEQ ID NO: 2, SEQ ID NO: 4 or SEQ ID NO: 5, and preferably has an amino acid sequence in which x/y is 6.2% or more, in a case where in all REPs included in a sequence excluding a sequence from a (A)ₙ motif located the most C-terminal side to the C-terminal of the domain sequence from the domain sequence, the total number of amino acid residues contained in a region where an average value of hydropathy indices of four consecutive amino acid residues is 2.6 or more is set as x, and the total number of amino acid residues contained in the sequence excluding the sequence from the (A)ₙ motif located the most C-terminal side to the C-terminal of the domain sequence from the domain sequence is set as y.

The above-mentioned modified fibroin may include a tag sequence at either or both of the N-terminal and C-terminal. This makes it possible to isolate, immobilize, detect and visualize the modified fibroin.

The tag sequence may be, for example, an affinity tag utilizing specific affinity (binding property, affinity) with another molecule. As a specific example of the affinity tag, a histidine tag (His tag) can be mentioned. The His tag is a short peptide in which about 4 to 10 histidine residues are arranged and has a property of specifically binding to a metal ion such as nickel, so it can be used for isolation of modified fibroin by chelating metal chromatography. A specific example of the tag sequence may be an amino acid sequence set forth in SEQ ID NO: 5 (amino acid sequence including His tag).

In addition, a tag sequence such as glutathione-S-transferase (GST) that specifically binds to glutathione or a maltose binding protein (MBP) that specifically binds to maltose can also be used.

Further, an "epitope tag" utilizing an antigen-antibody reaction can also be used. By adding a peptide (epitope) showing antigenicity as a tag sequence, an antibody against the epitope can be bound. Examples of the epitope tag include an HA (peptide sequence of hemagglutinin of influenza virus) tag, a myc tag, and a FLAG tag. The modified fibroin can easily be purified with high specificity by utilizing an epitope tag.

It is also possible to use a tag sequence which can be cleaved with a specific protease. By treating a protein adsorbed through the tag sequence with protease, it is also possible to recover the modified fibroin cleaved from the tag sequence.

A more specific example of the modified fibroin including a tag sequence may be a modified fibroin including (iii) an amino acid sequence set forth in SEQ ID NO: 8, SEQ ID NO: 10 or SEQ ID NO: 11, or (iv) an amino acid sequence having 90% or more sequence identity with the amino acid sequence set forth in SEQ ID NO: 8, SEQ ID NO: 10 or SEQ ID NO: 11.

The amino acid sequences set forth in SEQ ID NOs: 8, 10 and 11 are obtained by adding the amino acid sequence set forth in SEQ ID NO: 6 (including a His tag) to the N-terminals of the amino acid sequences set forth in SEQ ID NOs: 2, 4 and 5, respectively.

The modified fibroin of (iii) may consist of an amino acid sequence set forth in SEQ ID NO: 8, SEQ ID NO: 10 or SEQ ID NO: 11.

The modified fibroin of (iv) includes an amino acid sequence having 90% or more sequence identity with the amino acid sequence set forth in SEQ ID NO: 8, SEQ ID NO: 10 or SEQ ID NO: 11. The modified fibroin of (iv) is also a protein including a domain sequence represented by Formula 1: [(A)ₙ motif-REP]ₘ. The sequence identity is preferably 95% or more.

The modified fibroin of (iv) preferably has 90% or more sequence identity with the amino acid sequence set forth in SEQ ID NO: 8, SEQ ID NO: 10 or SEQ ID NO: 11, and preferably has an amino acid sequence in which x/y is 6.2% or more, in a case where in all REPs included in a sequence excluding a sequence from (A)ₙ motif located the most C-terminal side to the C-terminal of the domain sequence from the domain sequence, the total number of amino acid residues contained in a region where an average value of hydropathy indices of four consecutive amino acid residues is 2.6 or more is set as x, and the total number of amino acid residues contained in the sequence excluding the sequence from the (A)ₙ motif located the most C-terminal side to the C-terminal of the domain sequence from the domain sequence is set as y.

The above-mentioned modified fibroin may include a secretory signal for releasing the protein produced in the recombinant protein production system to the outside of a host. The sequence of the secretory signal can be appropriately set depending on the type of the host.

### [Nucleic acid]

The nucleic acid according to the present invention encodes the modified fibroin according to the present invention. Specific examples of the nucleic acid include nucleic acids encoding a modified fibroin including an amino acid sequence set forth in SEQ ID NO: 2, SEQ ID NO: 4 or SEQ ID NO: 5, or a protein or the like having an amino acid sequence (tag sequence) set forth in SEQ ID NO: 6 attached to either or both of the N-terminal and C-terminal of these amino acid sequences.

A nucleic acid according to an embodiment is a nucleic acid that hybridizes with a complementary strand of the nucleic acid that encodes the modified fibroin according to the present invention under stringent conditions and includes a domain sequence represented by Formula 1: [(A)ₙ motif-REP]ₘ in which x/y is 6.2% or more, in a case where in all REPs included in a sequence excluding a sequence from a (A)ₙ motif located the most C-terminal side to the C-terminal of the domain sequence from the domain sequence, the total number of amino acid residues contained in a region where an average value of hydropathy indices of four consecutive amino acid residues is 2.6 or more is set as x, and the total number of amino acid residues contained in the sequence excluding the sequence from the (A)ₙ motif located the most C-terminal side to the C-terminal of the domain sequence from the domain sequence is set as y.

The term "stringent conditions" refers to conditions under which a so-called specific hybrid is formed and a non-specific hybrid is not formed. The "stringent conditions" may be any of low stringent conditions, moderately stringent conditions and highly stringent conditions. The low stringent conditions mean that hybridization occurs only in the case where there is at least 85% or more identity between the sequences, and include, for example, conditions of hybridization at 42°C using 5×SSC containing 0.5% SDS. The moderately stringent conditions mean that hybridization occurs only in the case where there is at least 90% or more identity between the sequences, and include, for example, conditions of hybridization at 50°C using 5×SSC containing 0.5% SDS. The highly stringent conditions mean that hybridization occurs only in the case where there is at least 95% or more identity between the sequences, and include, for example, conditions of hybridization at 60°C using 5×SSC containing 0.5% SDS.

### [Host and expression vector]

An expression vector according to the present invention has a nucleic acid sequence according to the present invention and one or a plurality of regulatory sequences operably linked thereto. The regulatory sequence is a sequence (for example, a promoter, an enhancer, a ribosome binding sequence, or a transcription termination sequence) that controls the expression of a recombinant protein in a host, and can be appropriately selected depending on the type of the host. The type of the expression vector such as a plasmid vector, a viral vector, a cosmid vector, a fosmid vector, or an artificial chromosome vector can be appropriately selected depending on the type of the host.

The host according to the present invention is a host which has been transformed with the expression vector according to the present invention. Both prokaryotes and eukaryotes such as yeast, filamentous fungi, insect cells, animal cells, and plant cells can be suitably used as hosts.

As the expression vector, an expression vector which can autonomously replicate in a host cell or can be incorporated into a chromosome of a host and which contains a promoter at a position capable of transcribing the nucleic acid according to the present invention is suitably used.

In the case where a prokaryote such as a bacterium is used as a host, the expression vector according to the present invention is preferably a vector which is capable of autonomous replication in the prokaryote and at the same time includes a promoter, a ribosome binding sequence, a nucleic acid according to the present invention and a transcription termination sequence. A gene that controls a promoter may be included.

Examples of the prokaryote include microorganisms belonging to the genus *Escherichia, Brevibacillus, Serratia*, *Bacillus*, *Microbacterium, Brevibacterium, Corynebacterium* and *Pseudomonas.*

Examples of microorganisms belonging to the genus *Escherichia* include *Escherichia coli* BL21 (Novagen, Inc.), *Escherichia coli* BL21 (DE3) (Life Technologies Corporation), *Escherichia coli* BLR (DE3) (Merck KGaA), *Escherichia coli* DH1, *Escherichia coli* GI698, *Escherichia coli* HB101, *Escherichia coli* JM109, *Escherichia coli* K5 (ATCC 23506), *Escherichia coli* KY3276, *Escherichia coli* MC1000, *Escherichia coli* MG1655 (ATCC 47076), *Escherichia coli* No. 49, *Escherichia coli* Rosetta (DE3) (Novagen, Inc.), *Escherichia coli* TB1, *Escherichia coli* Tuner (Novagen, Inc.), *Escherichia coli* Tuner (DE3) (Novagen, Inc.), *Escherichia coli* W1485, *Escherichia coli* W3110 (ATCC 27325), *Escherichia coli* XL1-Blue, and *Escherichia coli* XL2-Blue.

Examples of microorganisms belonging to the genus *Brevibacillus* include *Brevibacillus agri*, *Brevibacillus borstelensis*, *Brevibacillus centrosporus, Brevibacillus formosus, Brevibacillus invocatus, Brevibacillus laterosporus*, *Brevibacillus limnophilus*, *Brevibacillus parabrevis, Brevibacillus reuszeri, Brevibacillus thermoruber*, *Brevibacillus brevis* 47 (FERM BP-1223), *Brevibacillus brevis* 47K (FERM BP-2308), *Brevibacillus brevis* 47-5 (FERM BP-1664), *Brevibacillus brevis* 47-5Q (JCM 8975), *Brevibacillus choshinensis* HPD31 (FERM BP-1087), *Brevibacillus choshinensis* HPD31-S (FERM BP-6623), *Brevibacillus choshinensis* HPD31-OK (FERM BP-4573), and *Brevibacillus choshinensis* SP3 strain (manufactured by Takara Bio, Inc.).

Examples of microorganisms belonging to the genus *Serratia* include *Serratia liquefacience* ATCC 14460, *Serratia entomophila*, *Serratia ficaria*, *Serratia fonticola*, *Serratia grimesii, Serratia proteamaculans*, *Serratia odorifera*, *Serratia plymuthica*, and *Serratia rubidaea.*

Examples of microorganisms belonging to the genus *Bacillus* include *Bacillus subtilis* and *Bacillus amyloliquefaciens.*

Examples of microorganisms belonging to the genus *Microbacterium* include *Microbacterium ammoniaphilum* ATCC 15354.

Examples of microorganisms belonging to the genus *Brevibacterium* include *Brevibacterium divaricatum (Corynebacterium glutamicum*) ATCC 14020, *Brevibacterium flavum (Corynebacterium glutamicum* ATCC 14067) ATCC 13826, ATCC 14067, *Brevibacterium immariophilum* ATCC 14068, *Brevibacterium lactofermentum (Corynebacterium glutamicum* ATCC 13869) ATCC 13665, ATCC 13869, *Brevibacterium roseum* ATCC 13825, *Brevibacterium saccharolyticum* ATCC 14066, *Brevibacterium tiogenitalis* ATCC 19240, *Brevibacterium album* ATCC 15111, and *Brevibacterium cerinum* ATCC 15112.

Examples of microorganisms belonging to the genus *Corynebacterium* include *Corynebacterium ammoniagenes* ATCC 6871, ATCC 6872, *Corynebacterium glutamicum* ATCC 13032, *Corynebacterium glutamicum* ATCC 14067, *Corynebacterium acetoacidophilum* ATCC 13870, *Corynebacterium-acetoglutamicum* ATCC 15806, *Corynebacterium alkanolyticum* ATCC 21511, *Corynebacterium callunae* ATCC 15991, *Corynebacterium glutamicum* ATCC 13020, ATCC 13032, ATCC 13060, *Corynebacterium lilium* ATCC 15990, *Corynebacterium melassecola* ATCC 17965, *Corynebacterium thermoaminogenes* AJ12340 (FERM BP-1539), and *Corynebacterium herculis* ATCC 13868.

Examples of microorganisms belonging to the genus *Pseudomonas* include *Pseudomonas putida, Pseudomonas fluorescens, Pseudomonas brassicacearum, Pseudomonas fulva*, and *Pseudomonas* sp. D-0110.

As a method for introducing an expression vector into the foregoing host cell, any method can be used as long as it introduces DNA into the host cell. Examples thereof include a method using calcium ions [Proc. Natl. Acad. Sci. USA, 69, 2110 (1972)], a protoplast method (Japanese Unexamined Patent Publication No. S63-248394), or a method described in Gene, 17, 107 (1982) or Molecular & General Genetics, 168, 111 (1979).

Transformation of microorganisms belonging to the genus *Brevibacillus* can be carried out, for example, by the method of Takahashi et al. (J. Bacteriol., 1983, 156: 1130-1134), the method of Takagi et al. (Agric. Biol. Chem., 1989, 53: 3099-3100), or the method of Okamoto et al. (Biosci. Biotechnol. Biochem., 1997, 61: 202-203).

Examples of the vector into which the nucleic acid according to the present invention is introduced (hereinafter, simply referred to as "vector") include pBTrp2, pBTacl, and pBTac2 (all commercially available from Boehringer Mannheim GmbH), pKK233-2 (manufactured by Pharmacia Corporation), pSE280 (manufactured by Invitrogen Corporation), pGEMEX-1 (manufactured by Promega Corporation), pQE-8 (manufactured by QIAGEN Corporation), pKYP10 (Japanese Unexamined Patent Publication No. S58-110600), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene Corporation), pTrs30 [constructed from *Escherichia coli* JM109/pTrS30 (FERM BP-5407)], pTrs32 [constructed from *Escherichia coli* JM109/pTrS32 (FERM BP-5408)], pGHA2 [constructed from *Escherichia coli* IGHA2 (FERM B-400), Japanese Unexamined Patent Publication No. S60-221091], pGKA2 [constructed from Escherichia coli IGKA 2 (FERM BP-6798), Japanese Unexamined Patent Publication No. 60-221091], pTerm2 (US 4686191, US 4939094, US 5160735), pSupex, pUB110, pTP5, pC194, pEG400 [J. Bacteriol., 172, 2392 (1990)], pGEX (manufactured by Pharmacia Corporation), and pET systems (manufactured by Novagen, Inc.).

In the case where *Escherichia coli* is used as a host, pUC18, pBluescriptII, pSupex, pET22b, pCold, or the like can be mentioned as a suitable vector.

Specific examples of vectors suitable for microorganisms belonging to the genus *Brevibacillus* include pUB110 or pHY500 (Japanese Unexamined Patent Publication No. H2-31682), pNY700 (Japanese Unexamined Patent Publication No. H4-278091), pHY4831 (J. Bacteriol., 1987, 1239-1245), pNU200 (UDAKA Shigezou, Journal of the Agricultural Chemical Society of Japan, 1987, 61: 669-676), pNU100 (Appl. Microbiol. Biotechnol., 1989, 30: 75-80), pNU211 (J. Biochem., 1992, 112: 488-491), pNU211R2L5 (Japanese Unexamined Patent Publication No. H7-170984), pNH301 (Appl. Environ. Microbiol., 1992, 58: 525-531), pNH326, pNH400 (J. Bacteriol., 1995, 177: 745-749), and pHT210 (Japanese Unexamined Patent Publication No. H6-133782), pHT110R2L5 (Appl. Microbiol. Biotechnol., 1994, 42: 358-363), which are known as *Bacillus subtilis* vectors; and pNCO2 (Japanese Unexamined Patent Publication No. 2002-238569) which is a shuttle vector between *Escherichia coli* and a microorganism belonging to the genus *Brevibacillus.*

The promoter is not limited as long as it functions in a host cell. Examples thereof include promoters derived from *Escherichia coli* or phage such as a trp promoter (Ptrp), a lac promoter, a PL promoter, a PR promoter, and a T7 promoter. Also, promoters artificially designed and modified, such as a promoter (Ptrp×2) in which two Ptrp are connected in series, a tac promoter, a lacT7 promoter, and a let I promoter, can also be used.

It is preferable to use a plasmid in which the distance between the Shine-Dalgarno sequence, which is a ribosome binding sequence, and the initiation codon is adjusted to an appropriate distance (for example, 6 to 18 bases). In the expression vector according to the present invention, a transcription termination sequence is not always necessary for the expression of the nucleic acid according to the present invention, but it is preferable to arrange a transcription termination sequence immediately below a structural gene.

Examples of eukaryotic hosts include yeast, filamentous fungi (mold and the like), and insect cells.

Examples of the yeast include yeasts belonging to the genus *Saccharomyces, Schizosaccharomyces, Kluyveromyces, Trichosporon, Schwanniomyces, Pichia, Candida, Yarrowia, Hansenula*, and the like. More specific examples of the yeast include Saccharomyces cerevisiae, *Schizosaccharomyces pombe, Kluyveromyces lactis*, *Kluyveromyces marxianus, Trichosporon pullulans*, *Schwanniomyces alluvius*, *Schwanniomyces occidentalis, Candida utilis, Pichia pastoris, Pichia angusta*, *Pichia methanolica*, *Pichia polymorpha*, *Pichia stipitis, Yarrowia lipolytica*, and *Hansenula polymorpha.*

It is preferred that the expression vector in the case where yeast is used as a host cell usually include an origin of replication (in the case where amplification in a host is required), a selection marker for propagation of the vector in *Escherichia coli,* a promoter and a terminator for recombinant protein expression in yeast, and a selection marker for yeast.

In the case where the expression vector is a non-integrating vector, it is preferable to further include an autonomously replicating sequence (ARS). This makes it possible to improve the stability of the expression vectors in cells (Myers, A. M., et al. (1986) Gene 45: 299-310).

Examples of the vector in the case where yeast is used as a host include YEP13 (ATCC 37115), YEp24 (ATCC 37051), YCp50 (ATCC 37419), YIp, pHS19, pHS15, pA0804, pHIL3Ol, pHIL-S1, pPIC9K, pPICZα, pGAPZα, and pPICZ B.

The promoter is not limited as long as it can be expressed in yeast. Examples of the promoter include a promoter of glycolytic genes such as hexose kinase, a PHO5 promoter, a PGK promoter, a GAP promoter, an ADH promoter, a gal 1 promoter, a gal 10 promoter, a heat shock polypeptide promoter, an MFα1 promoter, a CUP 1 promoter, a pGAP promoter, a pGCW14 promoter, an AOX1 promoter, and an MOX promoter.

As a method for introducing an expression vector into yeast, any method can be used as long as it introduces DNA into yeast. Examples thereof include an electroporation method (Methods Enzymol., 194, 182 (1990)), a spheroplast method (Proc. Natl. Acad. Sci., USA, 81, 4889 (1984)), a lithium acetate method (J. Bacteriol., 153, 163 (1983)), and a method described in Proc. Natl. Acad. Sci. USA, 75, 1929 (1978).

Examples of filamentous fungi include fungi belonging to the genus *Acremonium*, *Aspergillus*, *Ustilago*, *Trichoderma, Neurospora*, *Fusarium, Humicola*, *Penicillium*, *Myceliophtora*, *Botryts, Magnaporthe, Mucor, Metarhizium, Monascus, Rhizopus,* and *Rhizomucor.*

Specific examples of filamentous fungi include *Acremonium alabamense*, *Acremonium cellulolyticus*, *Aspergillus aculeatus*, *Aspergillus awamori, Aspergillus oryzae, Aspergillus sake, Aspergillus sojae, Aspergillus tubigensis, Aspergillus niger, Aspergillus nidulans*, *Aspergillus parasiticus, Aspergillus ficuum, Aspergillus phoeicus, Aspergillus foetidus, Aspergillus flavus, Aspergillus fumigatus, Aspergillus japonicus, Trichoderma viride, Trichoderma harzianum, Trichoderma reseei*, *Chrysosporium lucknowense, Thermoascus*, *Sporotrichum, Sporotrichum cellulophilum*, *Talaromyces*, *Thielavia terrestris, Thielavia*, *Neurospora crassa, Fusarium oxysporus, Fusarium graminearum, Fusarium venenatum, Humicola insolens*, *Penicillium chrysogenum, Penicillium camemberti*, *Penicillium canescens, Penicillium emersonii, Penicillium funiculosum, Penicillium griseoroseum*, *Penicillium purpurogenum*, *Penicillium roqueforti, Myceliophtaora thermophilum, Mucor ambiguus*, *Mucor circinelloides, Mucor fragilis, Mucor hiemalis, Mucor inaequisporus, Mucor oblongiellipticus*, *Mucor racemosus*, *Mucor recurvus*, *Mocor saturninus*, *Mocor subtilissmus*, *Ogataea polymorpha*, *Phanerochaete chrysosporium*, *Rhizomucor miehei*, *Rhizomucor pusillus*, and *Rhizopus arrhizus.*

The promoter in the case where the host is a filamentous fungus may be any one of a gene related to a glycolytic system, a gene related to constitutive expression, an enzyme gene related to hydrolysis, and the like. Specific examples thereof include amyB, glaA, agdA, glaB, TEF1, xynF1 tannase gene, No. 8AN, gpdA, pgkA, enoA, melO, sodM, catA, and catB.

Introduction of the expression vector into filamentous fungi can be carried out by a conventionally known method. Examples thereof include the method of Cohen et al. (calcium chloride method) [Proc. Natl. Acad. Sci. USA, 69: 2110 (1972)], a protoplast method [Mol. Gen. Genet., 168:111 (1979)], a competent method [J. Mol. Biol., 56: 209 (1971)], and an electroporation method.

Insect cells include, for example, lepidopteran insect cells, more specifically insect cells derived from *Spodoptera frugiperda* such as Sf9 and Sf21, and insect cells derived from *Trichoplusia ni* such as High 5.

Examples of the vector in the case where an insect cell is used as a host include baculoviruses such as *Autographa californica* nuclear polyhedrosis virus which is a virus that infects insects belonging to the family *Noctuidae* (Baculovirus Expression Vectors, A Laboratory Manual, W. H. Freeman and Company, New York (1992)).

In the case where an insect cell is used as a host, a polypeptide can be expressed by the method described in, for example, Current Protocols in Molecular Biology, Baculovirus Expression Vectors, A Laboratory Manual, W. H. Freeman and Company, New York (1992), or Bio/Technology, 6, 47 (1988). That is, a recombinant gene transfer vector and a baculovirus are co-introduced into an insect cell to obtain a recombinant virus (expression vector) in an insect cell culture supernatant, and then the recombinant virus is further infected into an insect cell, whereby the polypeptide can be expressed. Examples of the gene transfer vector used in the above method include pVL1392, pVL1393, and pBlueBacIII (all manufactured by Invitorogen Corporation).

As a method for co-introducing a recombinant gene transfer vector and a baculovirus into an insect cell for constructing the recombinant virus, for example, a calcium phosphate method (Japanese Unexamined Patent Publication No. H2-227075), a lipofection method (Proc. Natl. Acad. Sci. USA, 84, 7413 (1987)), or the like can be mentioned.

The recombinant vector according to the present invention preferably further contains a selection marker gene for selecting a transformant. For example, in *Escherichia coli,* resistance genes for various drugs such as tetracycline, ampicillin, and kanamycin can be used as selection marker genes. A recessive selection marker capable of complementing a genetic mutation involved in auxotrophy can also be used. In yeast, a resistance gene for geneticin can be used as a selection marker gene, and a gene complementing a genetic mutation involved in auxotrophy, or a selection marker such as LEU2, URA3, TRP1, or HIS3 can also be used. Examples of the selection marker gene for filamentous fungi include a marker gene selected from the group consisting of niaD (Biosci. Biotechnol. Biochem., 59, 1795-1797 (1995)), argB (Enzyme Microbiol Technol, 6, 386-389, (1984)), sC (Gene, 84, 329-334, (1989)), ptrA (BiosciBiotechnol Biochem, 64, 1416-1421, (2000)), pyrG (BiochemBiophys Res Commun, 112, 284-289, (1983)), amdS (Gene, 26, 205-221, (1983)), aureobasidin resistance gene (Mol Gen Genet, 261, 290-296, (1999)), benomyl resistance gene (Proc Natl Acad Sci USA, 83, 4869-4873, (1986)) and hygromycin resistance gene (Gene, 57, 21-26, (1987)), and a leucine auxotrophy-complementing gene. Further, in the case where the host is an auxotrophic mutant strain, a wild-type gene complementing the auxotrophy can also be used as a selection marker gene.

The selection of the host transformed with the expression vector according to the present invention can be carried out by plaque hybridization and colony hybridization using a probe that selectively binds to the nucleic acid according to the present invention. As the probe, it is possible to use a probe obtained by modifying a partial DNA fragment amplified by a PCR method based on sequence information of the nucleic acid according to the present invention with a radioisotope or digoxigenin.

### (Production of modified fibroin)

In the host transformed with the expression vector according to the present invention, the modified fibroin according to the present invention can be produced by expressing the nucleic acid according to the present invention. As for the expression method, secretory production, fusion protein expression, or the like, in addition to direct expression, can be carried out according to the method described in Molecular Cloning, 2nd edition. In the case where it is expressed by yeast, an animal cell, or an insect cell, a modified fibroin can be obtained as a polypeptide to which a sugar or sugar chain is added.

The modified fibroin according to the present invention can be produced, for example, by culturing a host transformed with the expression vector according to the present invention in a culture medium, producing and accumulating the modified fibroin according to the present invention in the culture medium, and then collecting the modified fibroin from the culture medium. The method for culturing the host according to the present invention in a culture medium can be carried out according to a method commonly used for culturing a host.

In the case where the host according to the present invention is a prokaryote such as *Escherichia coli* or a eukaryote such as yeast, any of a natural medium and a synthetic medium may be used as a culture medium of the host according to the present invention as long as it contains a carbon source, a nitrogen source, inorganic salts and the like which can be assimilated by the host and it is capable of efficiently culturing the host.

As the carbon source, any carbon source that can be assimilated by the host may be used. Examples of the carbon source that can be used include carbohydrates such as glucose, fructose, sucrose, and molasses, starch and starch hydrolyzates containing them, organic acids such as acetic acid and propionic acid, and alcohols such as ethanol and propanol.

Examples of the nitrogen source that can be used include ammonium salts of inorganic or organic acids such as ammonia, ammonium chloride, ammonium sulfate, ammonium acetate and ammonium phosphate, other nitrogen-containing compounds, peptone, meat extract, yeast extract, corn steep liquor, casein hydrolyzate, soybean cake and soybean cake hydrolyzate, various fermented microbial cells and digested products thereof.

Examples of the inorganic salt that can be used include potassium dihydrogen phosphate, dipotassium phosphate, magnesium phosphate, magnesium sulfate, sodium chloride, ferrous sulfate, manganese sulfate, copper sulfate, and calcium carbonate.

Culture of a prokaryote such as *Escherichia coli* or a eukaryote such as yeast can be carried out under aerobic conditions such as shaking culture or deep aeration stirring culture. The culture temperature is, for example, 15°C to 40°C. The culture time is usually 16 hours to 7 days. It is preferable to maintain the pH of the culture medium during the culture at 3.0 to 9.0. The pH of the culture medium can be adjusted using an inorganic acid, an organic acid, an alkali solution, urea, calcium carbonate, ammonia, or the like.

In addition, antibiotics such as ampicillin and tetracycline may be added to the culture medium as necessary during the culture. In the case of culturing a microorganism transformed with an expression vector using an inducible promoter as a promoter, an inducer may be added to the medium as necessary. For example, in the case of culturing a microorganism transformed with an expression vector using a lac promoter, isopropyl-β-D-thiogalactopyranoside or the like is used, and in the case of culturing a microorganism transformed with an expression vector using a trp promoter, indole acrylic acid or the like may be added to the medium.

As a culture medium for insect cells, commonly used TNM-FH medium (manufactured by Pharmingen Inc.), Sf-900 II SFM medium (manufactured by Life Technologies Corporation), ExCell 400 and ExCell 405 (both manufactured by JRH Biosciences Inc.), Grace's Insect Medium (Nature, 195, 788 (1962)), and the like can be used.

Culture of insect cells can be carried out, for example, for a culture time of 1 to 5 days under conditions such as pH 6 to 7 of culture medium and culture temperature 25°C to 30°C. In addition, an antibiotic such as gentamicin may be added to the culture medium as necessary during the culture.

In the case where the host is a plant cell, the transformed plant cell may be directly cultured, or it may be differentiated into a plant organ and then cultured. As the culture medium for culturing a plant cell, for example, commonly used Murashige and Skoog (MS) medium, White medium, or a medium in which a plant hormone such as auxin or cytokinin is added to these media can be used.

Culture of animal cells can be carried out, for example, for a culture time of 3 to 60 days under conditions such as pH 5 to 9 of the culture medium and culture temperature 20°C to 40°C. In addition, an antibiotic such as kanamycin or hygromycin may be added to the medium as necessary during the culture.

As a method for producing a modified fibroin using a host transformed with the expression vector according to the present invention, there are a method for producing the modified fibroin in a host cell, a method for secreting the modified fibroin outside the host cell, and a method for producing the modified fibroin on the outer membrane of the host cell. Each of these methods can be selected by changing the host cell to be used and the structure of the modified fibroin to be produced.

For example, in the case where a modified fibroin is produced in the host cell or on the outer membrane of the host cell, the production method can be altered to actively secrete the modified fibroin outside the host cell according to the method of Paulson et al. (J. Biol. Chem., 264, 17619 (1989)), the method of Lowe et al. (Proc. Natl. Acad. Sci. USA, 86, 8227 (1989), Genes Develop., 4, 1288 (1990)), or the methods described in Japanese Unexamined Patent Publication No. H5-336963, International Publication No. WO94/23021, and the like. That is, the modified fibroin can be actively secreted outside the host cell by expressing the modified fibroin in a form in which a signal peptide is added to a polypeptide containing an active site of a modified fibroin using a gene recombination technique.

The modified fibroin produced by the host transformed with the expression vector according to the present invention can be isolated and purified by a method commonly used for protein isolation and purification. For example, in the case where the modified fibroin is expressed in a dissolved state in cells, the host cells are recovered by centrifugation after completion of the culture, suspended in an aqueous buffer solution, and then disrupted using an ultrasonicator, a French press, a Manton-Gaulin homogenizer, a Dyno-Mill, or the like to obtain a cell-free extract. From the supernatant obtained by centrifuging the cell-free extract, a purified preparation can be obtained by a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION HPA-75 (manufactured by Mitsubishi Kasei Kogyo Kabushiki Kaisha), an cation exchange chromatography method using a resin such as S-Sepharose FF (Pharmacia Corporation), a hydrophobic chromatography method using a resin such as butyl sepharose or phenyl sepharose, a gel filtration method using a molecular sieve, an affinity chromatography method, a chromatofocusing method, an electrophoresis method such as isoelectric focusing or the like, alone or in combination thereof.

As the chromatography, column chromatography using phenyl-TOYOPEARL (available from Tosoh Corporation), DEAE-TOYOPEARL (available from Tosoh Corporation), and Sephadex G-150 (available from Pharmacia Biotech Inc.) is preferably used.

In the case where the modified fibroin is expressed by the formation of an insoluble matter in the cell, similarly, the host cells are recovered, disrupted and centrifuged to recover the insoluble matter of the modified fibroin as a precipitated fraction. The recovered insoluble matter of the modified fibroin can be solubilized with a protein denaturing agent. After this operation, a purified preparation of modified fibroin can be obtained by the same isolation and purification method as described above.

In the case where a modified fibroin or a derivative in which a sugar chain has been added to the modified fibroin is secreted extracellularly, the modified fibroin or the derivative thereof can be recovered from the culture supernatant. That is, a culture supernatant is obtained by treating the culture by a technique such as centrifugation, and a purified preparation can be obtained from the culture supernatant by using the same isolation and purification method as described above.

### [Manufacturing method of molded article]

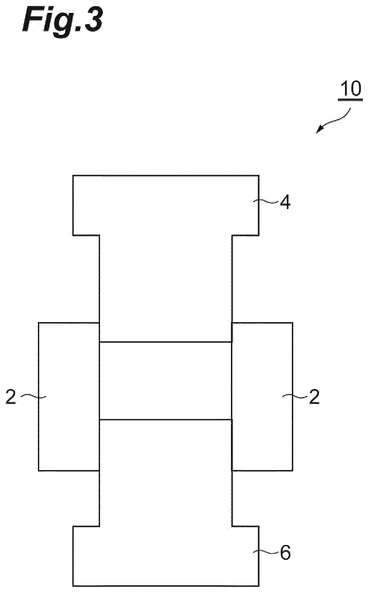

The molded article according to the present embodiment may be manufactured from a molding composition containing the modified fibroin according to the present embodiment using a press molding apparatus. FIG. 3 is a schematic cross-sectional diagram showing an embodiment of the press molding apparatus used for manufacturing a molded article. The press molding apparatus 10 shown in FIG. 3 includes a mold 2 capable of heating through which a through hole is formed, and an upper pin 4 and a lower pin 6 capable of moving up and down within the through hole of the mold 2. The molding composition containing the modified fibroin is introduced into the cavity formed by inserting the upper pin 4 or the lower pin 6 into the mold 2, and while heating the mold 2, the upper pin 4 and the lower pin 6, the composition is compressed, whereby the molded article can be obtained.

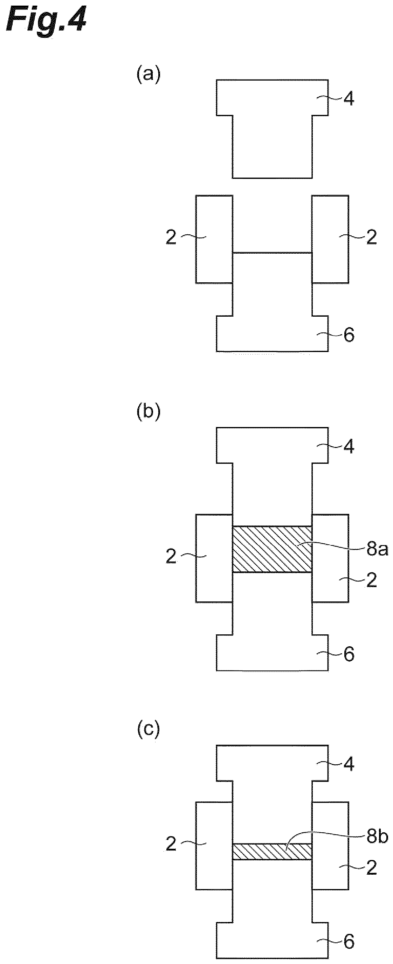

FIG. 4 is a process diagram schematically showing an embodiment of a method for manufacturing a molded article. FIG. 4(a) is a schematic cross-sectional diagram of the press molding apparatus before introducing the composition, FIG. 4(b) is a schematic cross-sectional diagram of the press molding apparatus immediately after introduction of the composition, and FIG. 4(c) is a schematic cross-sectional diagram of a press molding apparatus in a state where the composition is being heated and pressurized, or after the heating and pressurization. As shown in FIG. 4 (a), the composition is introduced into the through hole while only the lower pin 6 is inserted into the through hole of the mold 2, and as shown in FIG. 4 (b), the upper pin 4 is inserted and lowered into the mold 2, heating of the mold 2 is started, and the composition 8a before heating and pressurizing is heated and pressurized in the through hole. The upper pin 4 is lowered until a predetermined pressure is reached and the heating and pressurizing are continued until the composition reaches a predetermined temperature in the state shown in FIG. 4 (c), and the composition is continuously heated and pressurized until the composition 8b after heating and pressurization 8b is obtained. Thereafter, the temperature of the mold 2 is lowered using a cooler (for example, a spot cooler), and when the composition 8b reaches a predetermined temperature, the upper pin 4 or the lower pin 6 is pulled out from the mold 2, and the contents are taken out to obtain the molded article. Regarding the pressurization, the upper pin 4 may be lowered while fixing the lower pin 6, but both lowering of the upper pin 4 and ascending of the lower pin 6 may also be performed.

The heating is preferably carried out at 80°C to 300°C, more preferably at 100°C to 180°C, and still more preferably at 100°C to 130°C. The pressurization is preferably carried out at 5kN or more, more preferably at 10kN or more, and still more preferably at 20kN or more. Further, the time during which the treatment is continued after a predetermined heating and pressurizing condition has been fulfilled (temperature retention condition) is preferably 0 to 100 minutes, more preferably 1 to 50 minutes, and still more preferably 5 to 30 minutes.

The molding composition containing the modified fibroin may contain only modified fibroin or may further contain optional additives. By including an additive, a molding composition having various different physical properties can be produced. Examples of the additives include softeners, crystal nucleating agents, foaming agents, weathering agents, hydrolysis resistors, reducing agents, colorants, modifiers, stabilizers, lubricants, fillers, flame retardants, reinforcing agents, mold release agents, antistatic agents, and antioxidants.

Examples of the softening agent include high molecular weight alcohols such as glycerin and polyethylene glycol, and sugars such as starch, water, surfactants.

Examples of the crystal nucleating agents include kaolin, talc, montmorilloid, magnesium oxide, aluminum oxide, and other types of polyamides.

Examples of the foaming agent include sodium bicarbonate plus acid or alkali, acid-added aluminum powder, water-added isocyanate with, and the like.

Examples of the weathering agent include benzotriazole, benzophenone, triazine and the like can be mentioned.

Examples of the hydrolysis resistant agent include epoxy reagent, carbodiimide, isocyanate, and oxazoline.

### [Product]

The fibroin fiber formed from the modified fibroin according to the present invention can be applied as a fiber or a yarn to a woven fabric, a knitted fabric, a braided fabric, and a nonwoven fabric. Such a fibroin fiber can also be applied to high strength applications such as ropes, surgical sutures, flexible stops for electrical components, and physiologically active materials for implantation (for example, artificial ligament and aortic band).

The modified fibroin according to the present invention can also be applied to filaments, films, foams, spheres, nanofibrils, hydrogels, resins and equivalents thereof, which can be produced in accordance with the method described in Japanese Unexamined Patent Publication No. 2009-505668, Japanese Unexamined Patent Publication No. 2009-505668, Japanese Patent No. 5678283, Japanese Patent No. 4638735, or the like.

### Examples

Hereinafter, the present invention will be described more specifically with respect to Examples. However, the present invention is not limited to the following Examples.

### [(1) Synthesis of nucleic acid encoding modified fibroin and construction of expression vector]

Based on the nucleotide sequence and amino acid sequence of *Nephila clavipes* (GenBank Accession Number: P46804.1, GI: 1174415) which is naturally occurring fibroin, fibroins and modified fibroins having amino acid sequences set forth in SEQ ID NOs: 1 to 5 and 7 to 11 were designed.

The amino acid sequence set forth in SEQ ID NO: 1 is a sequence obtained by deleting alanine residues of an amino acid sequence in which the alanine residues in the (A)ₙ motif of the naturally occurring fibroin are consecutive so that the number of consecutive alanine residues is 5. The amino acid sequence set forth in SEQ ID NO: 7 is a sequence obtained by adding the amino acid sequence set forth in SEQ ID NO: 6 (tag sequence and hinge sequence) to the N-terminal of the amino acid sequence set forth in SEQ ID NO: 1 (Comparative Example 1).

The amino acid sequence set forth in SEQ ID NO: 2 is a sequence obtained by inserting an amino acid sequence consisting of three amino acid residues (VLI) at two sites for each REP with respect to the amino acid sequence set forth in SEQ ID NO: 1, and deleting a part of the amino acid on the N-terminal side such that the molecular weight thereof is set to be approximately the same as that of the amino acid sequence set forth in SEQ ID NO: 1. The amino acid sequence set forth in SEQ ID NO: 8 is a sequence obtained by adding the amino acid sequence set forth in SEQ ID NO: 6 (tag sequence and hinge sequence) to the N-terminal of the amino acid sequence set forth in SEQ ID NO: 2 (Example 1).

The amino acid sequence set forth in SEQ ID NO: 3 is a sequence obtained by inserting two alanine residues at the C-terminal side of each (A)ₙ motif of the amino acid sequence set forth in SEQ ID NO: 1 and further substituting a part of glutamine (Q) residues with serine (S) residues and deleting a part of amino acids on the N-terminal side such that the molecular weight thereof is set to be approximately the same as the molecular weight of SEQ ID NO: 1. The amino acid sequence set forth in SEQ ID NO: 9 is a sequence obtained by adding the amino acid sequence set forth in SEQ ID NO: 6 (tag sequence and hinge sequence) to the N-terminal of the amino acid sequence set forth in SEQ ID NO: 2. (Comparative Example 2)

The amino acid sequence set forth in SEQ ID NO: 4 is a sequence obtained by inserting an amino acid sequence consisting of three amino acid residues (VLI) at one site for each REP with respect to the amino acid sequence set forth in SEQ ID NO: 3. The amino acid sequence set forth in SEQ ID NO: 10 is a sequence obtained by adding the amino acid sequence set forth in SEQ ID NO: 6 (tag sequence and hinge sequence) to the N-terminal of the amino acid sequence set forth in SEQ ID NO: 4 (Example 2).

The amino acid sequence set forth in SEQ ID NO: 5 is a sequence obtained by inserting an amino acid sequence consisting of three amino acid residues (VLI) at two sites for each REP with respect to the amino acid sequence set forth in SEQ ID NO: 3. The amino acid sequence set forth in SEQ ID NO: 11 is a sequence obtained by adding the amino acid sequence set forth in SEQ ID NO: 6 (tag sequence and hinge sequence) to the N-terminal of the amino acid sequence set forth in SEQ ID NO: 5 (Example 3).

Each of nucleic acids encoding proteins having amino acid sequences set forth in SEQ ID NOs: 7 to 11 in which a His tag sequence and a hinge sequence (SEQ ID NO: 6) have been added to the N-terminal of each designed amino acid sequence set forth in SEQ ID NOs: 1 to 5 was synthesized. In the nucleic acid, an *NdeI* site was added to the 5' end and an EcoRI site was added downstream of the stop codon. These five kinds of nucleic acids were cloned into a cloning vector (pUC118). Thereafter, the same nucleic acid was cleaved by restriction enzyme treatment with *NdeI* and *EcoRI,* and then recombined into a protein expression vector pET-22b(+) to obtain an expression vector.

### [(2) Expression of protein]

*Escherichia coli* BLR (DE3) was transformed with a pET22b(+) expression vector including each of nucleic acids encoding proteins having the amino acid sequences set forth in SEQ ID NOs: 7 to 11. The transformed *Escherichia coli* was cultured in 2 mL of an LB medium containing ampicillin for 15 hours. The culture solution was added to 100 mL of a seed culture medium (Table 2) containing ampicillin so that the OD₆₀₀ was 0.005. The temperature of the culture solution was maintained at 30°C and the flask culture was carried out (for about 15 hours) until the OD₆₀₀ reached 5, thereby obtaining a seed culture solution.

**[Table 2]**

| Seed culture medium (per 1 L at the time of culture start) | |
|---|---|
| Glucose | 5g |
| KH₂PO₄ | 4g |
| K₂HPO₄ | 10g |
| Yeast Extract | 6g |

Ampicillin was added to a final concentration of 100 mg/L to prepare seed culture medium

The seed culture solution was added to a jar fermenter to which 500 ml of a production medium (Table 3) had been added so that the OD₆₀₀ was 0.05, and the transformed *Escherichia coli* was inoculated. The culture was carried out while controlling the culture solution temperature at 37°C and keeping the pH constant at 6.9. Further, the dissolved oxygen concentration in the culture solution was maintained at 20% of the dissolved oxygen saturation concentration.

**[Table 3]**

| Production medium (per 1 L at the time of culture start) | |
|---|---|
| Glucose | 12g |
| KH₂PO₄ | 9g |
| MgSO₄·7H₂O | 2.4g |
| Yeast Extract | 15g |
| FeSO₄·7H₂O | 40mg |
| MnSO₄·5H₂O | 40mg |
| CaCl₂·2H₂O | 40mg |
| GD-113 (antifoaming agent) | 0.1mL |

Immediately after glucose in the production medium was completely consumed, a feed solution (455 g/1L of glucose and 120 g/1L of Yeast Extract) was added at a rate of 1 ml/min. The culture was carried out while controlling the culture solution temperature at 37°C and keeping the pH constant at 6.9. Further, the dissolved oxygen concentration in the culture solution was maintained at 20% of the dissolved oxygen saturation concentration, and the culture was carried out for 20 hours. Thereafter, 1 M isopropyl-β-thiogalactopyranoside (IPTG) was added to the culture solution to a final concentration of 1 mM to induce the expression of the target protein. Twenty hours after addition of IPTG, the culture solution was centrifuged to recover the bacterial cells. SDS-PAGE was carried out using the bacterial cells prepared from the culture solution before the addition of IPTG and after the addition of IPTG, and the expression of the target protein was confirmed by the appearance of a band of a target protein size depending on the addition of IPTG.

### [(3) Purification of protein]

The bacterial cells recovered 2 hours after the addition of IPTG were washed with 20 mM Tris-HCl buffer solution (pH 7.4). The bacterial cells after washing were suspended in 20 mM Tris-HCl buffer solution (pH 7.4) containing about 1 mM PMSF, and the cells were disrupted with a high-pressure homogenizer (available from GEA Niro Soavi SpA). The disrupted cells were centrifuged to obtain a precipitate. The obtained precipitate was washed with 20 mM Tris-HCl buffer solution (pH 7.4) until high purity. The precipitate after washing was suspended in 8 M guanidine buffer solution (8 M guanidine hydrochloride, 10 mM sodium dihydrogen phosphate, 20 mM NaCl, 1 mM Tris-HCl, pH 7.0) so as to have a concentration of 100 mg/mL, and dissolved by stirring with a stirrer at 60°C for 30 minutes. After dissolution, dialysis was carried out with water using a dialysis tube (cellulose tube 36/32 manufactured by Sanko Junyaku Co., Ltd.). The white aggregated protein obtained after dialysis was recovered by centrifugation, the water content was removed with a freeze dryer, and the freeze-dried powder was recovered.

The degree of purification of the target protein in the freeze-dried powder thus obtained was confirmed by image analysis of polyacrylamide gel electrophoresis results of the powder using TotalLab (Nonlinear Dynamics Ltd.). As a result, the purity of each protein was about 85%. The production amount of each target protein calculated from the weight of the freeze-dried powder is shown in Tables 4 and 5 as relative values when the value of GEN495 (SEQ ID NO: 7: Comparative Example 1) is taken as 100.

### [(4) Manufacture of molded article]

1.35 g of each of the freeze-dried powders (hereinafter referred to as "samples") obtained in the above [(3) Purification of protein] was weighed out, and this sample was placed in the mold 2 of the press molding apparatus 10 shown in FIG. 3 (having a rectangular through hole with a cross section of 35 mm × 15 mm). At this time, samples were added so that the thickness became even. After introducing all the samples, heating of the mold 2 was started, and the upper pin 4 and the lower pin 6 were inserted into the through holes by using a hand press machine (NT-100H-V09, manufactured by NPa System Co., Ltd.) to pressurize the sample. At this time, the pressure applied to the sample was controlled to be 40 kN. When the temperature of the sample reached 200°C, heating was stopped and the sample was cooled with a spot cooler (TS-25 EP-1, manufactured by Trasco Nakayama Co., Ltd.). When the temperature of the sample reached 50°C, the sample was taken out and deburring was carried out to obtain a molded article having a rectangular parallelepiped shape of 35 mm × 15 mm × 2 mm.

### [(5) Measurement of bending strength]

The obtained molded article was allowed to stand for 1 day under a condition of 20°C and 65% in a thermo-hygrostat (Leck LHL-113, manufactured by espec), then using an autograph (AG-Xplus, manufactured by Shimadzu Corporation) and basket jig, a three point bending test was conducted. A load cell with a rated capacity of 50 kN was used. At this time, the distance between fulcrums of three point bending was fixed at 27 mm and the measurement speed was set to 1 mm/min. The size of the molded article was measured with a micro caliper. The results of the bending strength measurement test are shown in Table 4 as relative values when the value of GEN 495 (SEQ ID NO: 7: Comparative Example 1) is taken as 100.

**[Table 4]**

| | Name | Productivity (%) | Bending strength (%) | Region of 4 amino acid residues having an HI of 2.6 or more (%) |
|---|---|---|---|---|
| Comparative Example 1 | GEN495 | 100.0 | 100.0 | 0.00% |
| Example 1 | GEN819 | 129.1 | 168.8 | 14.09% |

**[Table 5]**

| | Name | Productivity (%) | Region of 4 amino acid residues having an HI of 2.6 or more (%) |
|---|---|---|---|
| Comparative Example 2 | GEN514 | 131.6 | 0.00% |
| Example 2 | GEN767 | 168.4 | 6.27% |
| Example 3 | GEN765 | 169.6 | 12.37% |

The modified fibroins having an amino acid sequence containing a region with locally high hydropathy index in REP showed remarkably improved productivity (Examples 1 to 3), and a molded article produced from the modified fibroins showed a remarkably improved bending strength (Example 1).

### Reference Signs List

2 ... mold, 4... upper pin, 6... lower pin, 8a ... composition before heating and pressurizing, 8b ... composition after heating and pressurizing, 10 ... press molding apparatus.

## Claims

1. A modified fibroin, comprising:
a domain sequence represented by Formula 1: [(A)ₙ motif-REP]ₘ,
wherein the domain sequence has an amino acid sequence containing a region with locally high hydropathy index equivalent to an amino acid sequence in which one or a plurality of amino acid residues in REP are substituted with amino acid residues with a high hydropathy index and/or one or a plurality of amino acid residues with a high hydropathy index are inserted into REP, as compared to naturally occurring fibroin.
[In Formula 1, (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, and a plurality of REPs may be the same amino acid sequence or different amino acid sequences.]

2. The modified fibroin according to claim 1, wherein the region with locally high hydropathy index is constituted by two to four consecutive amino acid residues.

3. The modified fibroin according to claim 1 or 2, wherein the amino acid residues with a high hydropathy index are selected from isoleucine (I), valine (V), leucine (L), phenylalanine (F), cysteine (C), methionine (M), and alanine (A).

4. A modified fibroin, comprising:
a domain sequence represented by Formula 1: [(A)ₙ motif-REP]ₘ,
wherein in a case where in all REPs included in a sequence excluding the sequence from the (A)ₙ motif located at the most C-terminal side to a C-terminal of the domain sequence from the domain sequence, the total number of amino acid residues contained in a region where an average value of hydropathy indices of four consecutive amino acid residues is 2.6 or more is set as x, and the total number of amino acid residues contained in the sequence excluding the sequence from the (A)ₙ motif located at the most the C-terminal side to the C-terminal of the domain sequence from the domain sequence is set as y, x/y is 6.2% or more.
[In Formula 1, (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, and a plurality of REPs may be the same amino acid sequence or different amino acid sequences.]

5. The modified fibroin according to any one of claims 1 to 4, in which the fibroin has, in addition to the amino acid sequence in which one or a plurality of amino acid residues in REP are substituted with amino acid residues with a high hydropathy index and/or one or a plurality of amino acid residues with a high hydropathy index are inserted into REP, an amino acid sequence corresponding to substitution, deletion, insertion and/or addition of one or a plurality of amino acid residues, as compared to naturally occurring fibroin.

6. The modified fibroin according to claim 5, wherein the naturally occurring fibroin is a fibroin derived from an insect or a spider.

7. The modified fibroin according to claim 5, wherein the naturally occurring fibroin is a major ampullate spider protein (MaSp) or minor ampullate spider protein (MiSp) of spiders.

8. A modified fibroin, comprising:
an amino acid sequence set forth in SEQ ID NO: 2, SEQ ID NO: 4 or SEQ ID NO: 5, or
an amino acid sequence having 90% or more sequence identity with the amino acid sequence set forth in SEQ ID NO: 2, SEQ ID NO: 4 or SEQ ID NO: 5.

9. The modified fibroin according to any one of claims 1 to 8, further comprising a tag sequence at either or both of an N-terminal and a C-terminal.

10. The modified fibroin according to claim 9, wherein the tag sequence includes an amino acid sequence set forth in SEQ ID NO: 6.

11. A modified fibroin, comprising:
an amino acid sequence set forth in SEQ ID NO: 8, SEQ ID NO: 10 or SEQ ID NO: 11, or
an amino acid sequence having 90% or more sequence identity with the amino acid sequence set forth in SEQ ID NO: 8, SEQ ID NO: 10 or SEQ ID NO: 11.

12. A nucleic acid encoding the modified fibroin according to any one of claims 1 to 11.

13. A nucleic acid that hybridizes with a complementary strand of the nucleic acid according to claim 12 under stringent conditions and encodes a modified fibroin including a domain sequence represented by Formula 1: [(A)ₙ motif-REP]ₘ.
[In Formula 1, (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, and a plurality of REPs may be the same amino acid sequence or different amino acid sequences.]

14. A nucleic acid having 90% or more sequence identity with the nucleic acid according to claim 13 and encoding a modified fibroin including a domain sequence represented by Formula 1: [(A)ₙ motif-REP]ₘ.
[In Formula 1, the (A)ₙ motif represents an amino acid sequence consisting of 4 to 20 amino acid residues and the number of alanine residues relative to the total number of amino acid residues in the (A)ₙ motif is 83% or more, REP represents an amino acid sequence consisting of 10 to 200 amino acid residues, m represents an integer of 10 to 300, a plurality of (A)ₙ motifs may be the same amino acid sequence or different amino acid sequences, and a plurality of REPs may be the same amino acid sequence or different amino acid sequences.]

15. An expression vector, comprising:
the nucleic acid sequence according to any one of claims 12 to 14; and
one or a plurality of regulatory sequences operably linked thereto.

16. The expression vector according to claim 15, which is a plasmid vector or a viral vector.

17. A host transformed with the expression vector according to claim 15 or 16.

18. The host according to claim 17, which is a prokaryote.

19. The host according to claim 18, wherein the prokaryote is a microorganism belonging to a genus selected from the group consisting of *Escherichia*, *Brevibacillus*, *Serratia*, *Bacillus*, *Microbacterium*, *Brevibacterium*, *Corynebacterium* and *Pseudomonas.*

20. The host according to claim 17, which is a eukaryote.

21. The host according to claim 20, wherein the eukaryote is a yeast, a filamentous fungus or an insect cell.

22. The host according to claim 21, wherein the yeast is a yeast belonging to a genus selected from the group consisting of *Saccharomyces, Schizosaccharomyces, Kluyveromyces, Trichosporon, Schwanniomyces*, *Pichia*, *Candida*, *Yarrowia* and *Hansenula.*

23. The host according to claim 22, wherein the yeast belonging to the genus *Saccharomyces* is *Saccharomyces cerevisiae*, the yeast belonging to the genus *Schizosaccharomyces* is *Schizosaccharomyces pombe*, and the yeast belonging to the genus *Kluyveromyces* is *Kluyveromyces lactis*, the yeast belonging to the genus *Trichosporon* is *Trichosporon pullulans*, the yeast belonging to the genus *Schwaniomyces* is *Schwanniomyces alluvius*, the yeast belonging to the genus *Pichia* is *Pichia pastoris*, the yeast belonging to the genus *Candida* is *Candida albicans*, the yeast belonging to the genus *Yarrowia* is *Yarrowia lipolytica*, and the yeast belonging to the genus *Hansenula* is *Hansenula polymorpha.*

24. The host according to claim 21, wherein the filamentous fungus is a filamentous fungus belonging to a genus selected from the group consisting of *Aspergillus*, *Penicillium* and *Mucor.*

25. The host according to claim 24, wherein the filamentous fungus belonging to the genus *Aspergillus* is *Aspergillus oryzae*, the filamentous fungus belonging to the genus *Penicillium* is *Penicillium chrysogenum*, and the filamentous fungus belonging to the genus *Mucor* is *Mucor fragilis.*

26. The host according to claim 21, wherein the insect cell is a lepidopteran insect cell.

27. The host according to claim 26, wherein the insect cell is an insect cell derived from *Spodoptera frugiperda* or an insect cell derived from *Trichoplusia ni.*

28. A product comprising the modified fibroin according to any one of claims 1 to 11 and selected from the group consisting of a fiber, a yarn, a filament, a film, a foam, a sphere, a nanofibril, a hydrogel, a resin and an equivalent thereof.


SEQUENCE LISTING

<110>  Spiber Inc.
       KOJIMA INDUSTRIES CORPORATION

<120>  Altered Fibroin

<130>  FP17-0358-00

<160>  11

<170>  PatentIn version 3.5

<210>  1
<211>  590
<212>  PRT
<213>  Artificial Sequence

<220>
<223>  Met-GEN495
<400>  1

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro

165                 170                 175

Gly Gln Tyr Gly Pro Tyr Ala Ser Ala Ala Ala Ala Ala Gly Gln Tyr
        180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  2
<211>  612
<212>  PRT
<213>  Artificial Sequence

<220>
<223>  Met-GEN819

<400>  2

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly

        35                  40                  45

Pro Gly Ser Ser Ala Ala Ala Ala Ala Gly Pro Gly Gln Tyr Val Leu
    50                  55                  60

Ile Gly Pro Gly Gln Gln Val Leu Ile Gly Pro Ser Ala Ser Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Gly Ser Tyr Gly Ser Val Leu Ile Gly Pro
        115                 120                 125

Gly Gln Gln Val Leu Ile Gly Pro Tyr Gly Ser Ala Ala Ala Ala Ala
    130                 135                 140

Gly Pro Gly Ser Gly Gln Tyr Gly Gln Gly Pro Tyr Gly Pro Gly Ala
145                 150                 155                 160

Ser Gly Pro Gly Gln Tyr Gly Pro Gly Gln Gln Gly Pro Ser Ala Ser
                165                 170                 175

Ala Ala Ala Ala Ala Gly Ser Gly Gln Gln Val Leu Ile Gly Pro Gly
            180                 185                 190

Gln Tyr Val Leu Ile Gly Pro Tyr Ala Ser Ala Ala Ala Ala Ala Gly
        195                 200                 205

Gln Tyr Gly Ser Gly Pro Gly Gln Gln Gly Pro Tyr Gly Pro Gly Gln
    210                 215                 220

Ser Gly Ser Gly Gln Gln Gly Pro Gly Gln Gln Gly Pro Tyr Ala Ser
225                 230                 235                 240

Ala Ala Ala Ala Ala Gly Pro Gly Gln Gln Val Leu Ile Gly Pro Tyr
                245                 250                 255

Val Leu Ile Gly Pro Gly Ser Ser Ala Ala Ala Ala Ala Gly Gln Tyr
            260                 265                 270

Gly Tyr Gly Pro Gly Gln Gln Gly Pro Tyr Gly Pro Gly Ala Ser Gly
        275                 280                 285

Gln Asn Gly Pro Gly Ser Gly Gln Tyr Gly Pro Gly Gln Gln Gly Pro
    290                 295                 300

Gly Gln Ser Ala Ala Ala Ala Ala Gly Pro Gly Gln Gln Val Leu Ile
305                 310                 315                 320

Gly Pro Tyr Val Leu Ile Gly Pro Gly Ala Ser Ala Ala Ala Ala Ala
                325                 330                 335

Gly Gln Tyr Gly Pro Gly Gln Gln Gly Pro Gly Gln Tyr Gly Pro Gly
            340                 345                 350

Ser Ser Gly Pro Gly Gln Gln Gly Pro Tyr Gly Pro Gly Ser Ser Ala
        355                 360                 365

Ala Ala Ala Ala Gly Ser Tyr Gly Pro Gly Gln Gln Val Leu Ile Gly
    370                 375                 380

Pro Tyr Val Leu Ile Gly Pro Gly Pro Ser Ala Ala Ala Ala Ala Gly
385                 390                 395                 400

Gln Tyr Gln Gln Gly Pro Gly Gln Gln Gly Pro Tyr Gly Pro Gly Ala
                405                 410                 415

Ser Gly Pro Gly Gln Gln Gly Pro Tyr Gly Pro Gly Ala Ser Ala Ala
            420                 425                 430

Ala Ala Ala Gly Pro Gly Gln Tyr Val Leu Ile Gly Pro Gly Gln Gln
        435                 440                 445

Val Leu Ile Gly Pro Ser Ala Ser Ala Ala Ala Ala Ala Gly Gln Tyr
    450                 455                 460

Gly Ser Gly Pro Gly Gln Tyr Gly Pro Tyr Gly Pro Gly Gln Ser Gly
465                 470                 475                 480

Pro Gly Ser Gly Gln Gln Gly Gln Gly Pro Tyr Gly Pro Gly Ala Ser
                485                 490                 495

Ala Ala Ala Ala Ala Gly Ser Tyr Gly Pro Gly Gln Gln Val Leu Ile
            500                 505                 510

Gly Pro Tyr Val Leu Ile Gly Pro Gly Pro Ser Ala Ala Ala Ala Ala
        515                 520                 525

Gly Pro Gly Ser Gly Gln Tyr Gly Pro Gly Ala Ser Gly Gln Asn Gly
    530                 535                 540

Pro Gly Ser Gly Gln Tyr Gly Pro Gly Gln Gln Gly Pro Gly Gln Ser
545                 550                 555                 560

Ala Ala Ala Ala Ala Gly Gln Tyr Gln Gln Val Leu Ile Gly Pro Gly
                565                 570                 575

Gln Gln Gly Pro Tyr Val Leu Ile Gly Pro Gly Ala Ser Ala Ala Ala
            580                 585                 590

Ala Ala Gly Pro Gly Ser Gly Gln Gln Val Leu Ile Gly Pro Gly Ala
        595                 600                 605

Ser Val Leu Ile
    610

<210>  3
<211>  565
<212>  PRT
<213>  Artificial Sequence

<220>
<223>  Met-GEN514

<400>  3

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
        35                  40                  45

Gln Gly Pro Gly Ser Ser Ala Ala Ala Ala Ala Ala Ala Gly Pro Gly
    50                  55                  60

Gln Tyr Gly Pro Gly Gln Gln Gly Pro Ser Ala Ser Ala Ala Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Ala Ala Gly Ser Tyr Gly Ser Gly Pro Gly
        115                 120                 125

Gln Gln Gly Pro Tyr Gly Ser Ala Ala Ala Ala Ala Ala Ala Gly Pro
    130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala
                165                 170                 175

Ala Ala Ala Ala Ala Gly Ser Gly Gln Gln Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
    210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
                245                 250                 255

Ala Ala Ala Ala Ala Ala Ala Gly Ser Tyr Gly Tyr Gly Pro Gly Gln
            260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
    290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
                325                 330                 335

Gly Pro Gly Gln Tyr Gly Pro Gly Ser Ser Gly Pro Gly Gln Gln Gly
            340                 345                 350

Pro Tyr Gly Pro Gly Ser Ser Ala Ala Ala Ala Ala Ala Ala Gly Ser
        355                 360                 365

Tyr Gly Pro Gly Gln Gln Gly Pro Tyr Gly Pro Gly Pro Ser Ala Ala
    370                 375                 380

Ala Ala Ala Ala Ala Gly Ser Tyr Gln Gln Gly Pro Gly Gln Gln Gly
385                 390                 395                 400

Pro Tyr Gly Pro Gly Ala Ser Gly Pro Gly Gln Gln Gly Pro Tyr Gly
                405                 410                 415

Pro Gly Ala Ser Ala Ala Ala Ala Ala Ala Ala Gly Pro Gly Gln Tyr
            420                 425                 430

Gly Pro Gly Gln Gln Gly Pro Ser Ala Ser Ala Ala Ala Ala Ala Ala
        435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
                485                 490                 495

Gly Gln Gln Gly Pro Tyr Gly Pro Gly Pro Ser Ala Ala Ala Ala Ala
            500                 505                 510

Ala Ala Gly Pro Gly Ser Gly Gln Tyr Gly Pro Gly Ala Ser Gly Gln
        515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565

<210>  4
<211>  603
<212>  PRT
<213>  Artificial Sequence

<220>
<223>  Met-GEN767

<400>  4
   Met His His His His His His Ser Ser Gly Ser Ser Gly Pro Gly Gln
   1               5                   10                  15
   Gln Gly Pro Tyr Gly Pro Gly Ala Ser Ala Ala Ala Ala Ala Ala Ala
               20                  25                  30
   Gly Ser Asn Gly Pro Gly Ser Gly Gln Gln Gly Pro Gly Gln Ser Gly
           35                  40                  45
   Gln Tyr Gly Pro Gly Gln Gln Gly Pro Gly Gln Gln Gly Pro Gly Ser
       50                  55                  60
   Ser Ala Ala Ala Ala Ala Ala Ala Gly Pro Gly Gln Tyr Val Leu Ile
   65                  70                  75                  80
   Gly Pro Gly Gln Gln Gly Pro Ser Ala Ser Ala Ala Ala Ala Ala Ala
                   85                  90                  95
   Ala Gly Pro Gly Ser Gly Gln Gln Gly Pro Gly Ala Ser Gly Gln Tyr
               100                 105                 110
   Gly Pro Gly Gln Gln Gly Pro Gly Gln Gln Gly Pro Gly Ser Ser Ala
           115                 120                 125
   Ala Ala Ala Ala Ala Ala Gly Ser Tyr Gly Ser Val Leu Ile Gly Pro
       130                 135                 140
   Gly Gln Gln Gly Pro Tyr Gly Ser Ala Ala Ala Ala Ala Ala Ala Gly
   145                 150                 155                 160
   Pro Gly Ser Gly Gln Tyr Gly Gln Gly Pro Tyr Gly Pro Gly Ala Ser
                   165                 170                 175
   Gly Pro Gly Gln Tyr Gly Pro Gly Gln Gln Gly Pro Ser Ala Ser Ala
               180                 185                 190
   Ala Ala Ala Ala Ala Ala Gly Ser Gly Gln Gln Val Leu Ile Gly Pro
           195                 200                 205
   Gly Gln Tyr Gly Pro Tyr Ala Ser Ala Ala Ala Ala Ala Ala Ala Gly
       210                 215                 220
   Ser Tyr Gly Ser Gly Pro Gly Gln Gln Gly Pro Tyr Gly Pro Gly Gln
   225                 230                 235                 240
   Ser Gly Ser Gly Gln Gln Gly Pro Gly Gln Gln Gly Pro Tyr Ala Ser
                   245                 250                 255
   Ala Ala Ala Ala Ala Ala Ala Gly Pro Gly Gln Gln Val Leu Ile Gly
               260                 265                 270
   Pro Tyr Gly Pro Gly Ser Ser Ala Ala Ala Ala Ala Ala Ala Gly Ser
           275                 280                 285
   Tyr Gly Tyr Gly Pro Gly Gln Gln Gly Pro Tyr Gly Pro Gly Ala Ser
       290                 295                 300
   Gly Gln Asn Gly Pro Gly Ser Gly Gln Tyr Gly Pro Gly Gln Gln Gly
   305                 310                 315                 320
   Pro Gly Pro Ser Ala Ala Ala Ala Ala Ala Ala Gly Pro Gly Gln Gln
                   325                 330                 335
   Val Leu Ile Gly Pro Tyr Gly Pro Gly Ala Ser Ala Ala Ala Ala Ala
               340                 345                 350
   Ala Ala Gly Ser Tyr Gly Pro Gly Gln Gln Gly Pro Gly Gln Tyr Gly
           355                 360                 365
   Pro Gly Ser Ser Gly Pro Gly Gln Gln Gly Pro Tyr Gly Pro Gly Ser
       370                 375                 380
   Ser Ala Ala Ala Ala Ala Ala Ala Gly Ser Tyr Gly Pro Gly Gln Gln
   385                 390                 395                 400
   Val Leu Ile Gly Pro Tyr Gly Pro Gly Pro Ser Ala Ala Ala Ala Ala
                   405                 410                 415
   Ala Ala Gly Ser Tyr Gln Gln Gly Pro Gly Gln Gln Gly Pro Tyr Gly
               420                 425                 430
   Pro Gly Ala Ser Gly Pro Gly Gln Gln Gly Pro Tyr Gly Pro Gly Ala
           435                 440                 445
   Ser Ala Ala Ala Ala Ala Ala Ala Gly Pro Gly Gln Tyr Val Leu Ile
       450                 455                 460
   Gly Pro Gly Gln Gln Gly Pro Ser Ala Ser Ala Ala Ala Ala Ala Ala
   465                 470                 475                 480
   Ala Gly Ser Tyr Gly Ser Gly Pro Gly Gln Tyr Gly Pro Tyr Gly Pro
                   485                 490                 495
   Gly Gln Ser Gly Pro Gly Ser Gly Gln Gln Gly Gln Gly Pro Tyr Gly
               500                 505                 510
   Pro Gly Ala Ser Ala Ala Ala Ala Ala Ala Ala Gly Ser Tyr Gly Pro
           515                 520                 525
   Gly Gln Gln Val Leu Ile Gly Pro Tyr Gly Pro Gly Pro Ser Ala Ala
       530                 535                 540
   Ala Ala Ala Ala Ala Gly Pro Gly Ser Gly Gln Tyr Gly Pro Gly Ala
   545                 550                 555                 560
   Ser Gly Gln Asn Gly Pro Gly Ser Gly Gln Tyr Gly Pro Gly Gln Gln
                   565                 570                 575
   Gly Pro Gly Pro Ser Ala Ala Ala Ala Ala Ala Ala Gly Pro Gly Ser
               580                 585                 590
   Gly Gln Gln Gly Pro Gly Ala Ser Val Leu Ile
           595                 600

<210>  5
<211>  619
<212>  PRT
<213>  Artificial Sequence

<220>
<223>  Met-GEN765

<400>  5

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
        35                  40                  45

Gln Gly Pro Gly Ser Ser Ala Ala Ala Ala Ala Ala Ala Gly Pro Gly
    50                  55                  60

Gln Tyr Val Leu Ile Gly Pro Gly Gln Gln Val Leu Ile Gly Pro Ser
65                  70                  75                  80

Ala Ser Ala Ala Ala Ala Ala Ala Ala Gly Pro Gly Ser Gly Gln Gln
                85                  90                  95

Gly Pro Gly Ala Ser Gly Gln Tyr Gly Pro Gly Gln Gln Gly Pro Gly
            100                 105                 110

Gln Gln Gly Pro Gly Ser Ser Ala Ala Ala Ala Ala Ala Ala Gly Ser
        115                 120                 125

Tyr Gly Ser Val Leu Ile Gly Pro Gly Gln Gln Val Leu Ile Gly Pro
    130                 135                 140

Tyr Gly Ser Ala Ala Ala Ala Ala Ala Ala Gly Pro Gly Ser Gly Gln
145                 150                 155                 160

Tyr Gly Gln Gly Pro Tyr Gly Pro Gly Ala Ser Gly Pro Gly Gln Tyr
                165                 170                 175

Gly Pro Gly Gln Gln Gly Pro Ser Ala Ser Ala Ala Ala Ala Ala Ala
            180                 185                 190

Ala Gly Ser Gly Gln Gln Val Leu Ile Gly Pro Gly Gln Tyr Val Leu
        195                 200                 205

Ile Gly Pro Tyr Ala Ser Ala Ala Ala Ala Ala Ala Ala Gly Ser Tyr
    210                 215                 220

Gly Ser Gly Pro Gly Gln Gln Gly Pro Tyr Gly Pro Gly Gln Ser Gly
225                 230                 235                 240

Ser Gly Gln Gln Gly Pro Gly Gln Gln Gly Pro Tyr Ala Ser Ala Ala
                245                 250                 255

Ala Ala Ala Ala Ala Gly Pro Gly Gln Gln Val Leu Ile Gly Pro Tyr
            260                 265                 270

Val Leu Ile Gly Pro Gly Ser Ser Ala Ala Ala Ala Ala Ala Ala Gly
        275                 280                 285

Ser Tyr Gly Tyr Gly Pro Gly Gln Gln Gly Pro Tyr Gly Pro Gly Ala
    290                 295                 300

Ser Gly Gln Asn Gly Pro Gly Ser Gly Gln Tyr Gly Pro Gly Gln Gln
305                 310                 315                 320

Gly Pro Gly Pro Ser Ala Ala Ala Ala Ala Ala Ala Gly Pro Gly Gln
                325                 330                 335

Gln Val Leu Ile Gly Pro Tyr Val Leu Ile Gly Pro Gly Ala Ser Ala
            340                 345                 350

Ala Ala Ala Ala Ala Ala Gly Ser Tyr Gly Pro Gly Gln Gln Gly Pro
        355                 360                 365

Gly Gln Tyr Gly Pro Gly Ser Ser Gly Pro Gly Gln Gln Gly Pro Tyr
    370                 375                 380

Gly Pro Gly Ser Ser Ala Ala Ala Ala Ala Ala Ala Gly Ser Tyr Gly
385                 390                 395                 400

Pro Gly Gln Gln Val Leu Ile Gly Pro Tyr Val Leu Ile Gly Pro Gly
                405                 410                 415

Pro Ser Ala Ala Ala Ala Ala Ala Ala Gly Ser Tyr Gln Gln Gly Pro
            420                 425                 430

Gly Gln Gln Gly Pro Tyr Gly Pro Gly Ala Ser Gly Pro Gly Gln Gln
        435                 440                 445

Gly Pro Tyr Gly Pro Gly Ala Ser Ala Ala Ala Ala Ala Ala Ala Gly
    450                 455                 460

Pro Gly Gln Tyr Val Leu Ile Gly Pro Gly Gln Gln Val Leu Ile Gly
465                 470                 475                 480

Pro Ser Ala Ser Ala Ala Ala Ala Ala Ala Ala Gly Ser Tyr Gly Ser
                485                 490                 495

Gly Pro Gly Gln Tyr Gly Pro Tyr Gly Pro Gly Gln Ser Gly Pro Gly
            500                 505                 510

Ser Gly Gln Gln Gly Gln Gly Pro Tyr Gly Pro Gly Ala Ser Ala Ala
        515                 520                 525

Ala Ala Ala Ala Ala Gly Ser Tyr Gly Pro Gly Gln Gln Val Leu Ile
    530                 535                 540

Gly Pro Tyr Val Leu Ile Gly Pro Gly Pro Ser Ala Ala Ala Ala Ala
545                 550                 555                 560

Ala Ala Gly Pro Gly Ser Gly Gln Tyr Gly Pro Gly Ala Ser Gly Gln
                565                 570                 575

Asn Gly Pro Gly Ser Gly Gln Tyr Gly Pro Gly Gln Gln Gly Pro Gly
            580                 585                 590

Pro Ser Ala Ala Ala Ala Ala Ala Ala Gly Pro Gly Ser Gly Gln Gln
        595                 600                 605

Val Leu Ile Gly Pro Gly Ala Ser Val Leu Ile
    610                 615

<210>  6

<211>  12
<212>  PRT
<213>  Artificial Sequence

<220>
<223>  HisTag

<400>  6

Met His His His His His His Ser Ser Gly Ser Ser
1               5                   10


<210>  7
<211>  601
<212>  PRT
<213>  Artificial Sequence

<220>
<223>  GEN495

<400>  7

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210>  8
<211>  623
<212>  PRT
<213>  Artificial Sequence

<220>
<223>  GEN819

<400>  8
   Met His His His His His His Ser Ser Gly Ser Ser Gly Pro Gly Gln
   1               5                   10                  15
   Gln Gly Pro Tyr Gly Pro Gly Ala Ser Ala Ala Ala Ala Ala Gly Gln
               20                  25                  30
   Asn Gly Pro Gly Ser Gly Gln Gln Gly Pro Gly Gln Ser Gly Gln Tyr
           35                  40                  45
   Gly Pro Gly Gln Gln Gly Pro Gly Gln Gln Gly Pro Gly Ser Ser Ala
       50                  55                  60
   Ala Ala Ala Ala Gly Pro Gly Gln Tyr Val Leu Ile Gly Pro Gly Gln
   65                  70                  75                  80
   Gln Val Leu Ile Gly Pro Ser Ala Ser Ala Ala Ala Ala Ala Gly Pro
                   85                  90                  95
   Gly Ser Gly Gln Gln Gly Pro Gly Ala Ser Gly Gln Tyr Gly Pro Gly
               100                 105                 110
   Gln Gln Gly Pro Gly Gln Gln Gly Pro Gly Ser Ser Ala Ala Ala Ala
           115                 120                 125
   Ala Gly Ser Tyr Gly Ser Val Leu Ile Gly Pro Gly Gln Gln Val Leu
       130                 135                 140
   Ile Gly Pro Tyr Gly Ser Ala Ala Ala Ala Ala Gly Pro Gly Ser Gly
   145                 150                 155                 160
   Gln Tyr Gly Gln Gly Pro Tyr Gly Pro Gly Ala Ser Gly Pro Gly Gln
                   165                 170                 175
   Tyr Gly Pro Gly Gln Gln Gly Pro Ser Ala Ser Ala Ala Ala Ala Ala
               180                 185                 190
   Gly Ser Gly Gln Gln Val Leu Ile Gly Pro Gly Gln Tyr Val Leu Ile
           195                 200                 205
   Gly Pro Tyr Ala Ser Ala Ala Ala Ala Ala Gly Gln Tyr Gly Ser Gly
       210                 215                 220
   Pro Gly Gln Gln Gly Pro Tyr Gly Pro Gly Gln Ser Gly Ser Gly Gln
   225                 230                 235                 240
   Gln Gly Pro Gly Gln Gln Gly Pro Tyr Ala Ser Ala Ala Ala Ala Ala
                   245                 250                 255
   Gly Pro Gly Gln Gln Val Leu Ile Gly Pro Tyr Val Leu Ile Gly Pro
               260                 265                 270
   Gly Ser Ser Ala Ala Ala Ala Ala Gly Gln Tyr Gly Tyr Gly Pro Gly
           275                 280                 285
   Gln Gln Gly Pro Tyr Gly Pro Gly Ala Ser Gly Gln Asn Gly Pro Gly
       290                 295                 300
   Ser Gly Gln Tyr Gly Pro Gly Gln Gln Gly Pro Gly Gln Ser Ala Ala
   305                 310                 315                 320
   Ala Ala Ala Gly Pro Gly Gln Gln Val Leu Ile Gly Pro Tyr Val Leu
                   325                 330                 335
   Ile Gly Pro Gly Ala Ser Ala Ala Ala Ala Ala Gly Gln Tyr Gly Pro
               340                 345                 350
   Gly Gln Gln Gly Pro Gly Gln Tyr Gly Pro Gly Ser Ser Gly Pro Gly
           355                 360                 365
   Gln Gln Gly Pro Tyr Gly Pro Gly Ser Ser Ala Ala Ala Ala Ala Gly
       370                 375                 380
   Ser Tyr Gly Pro Gly Gln Gln Val Leu Ile Gly Pro Tyr Val Leu Ile
   385                 390                 395                 400
   Gly Pro Gly Pro Ser Ala Ala Ala Ala Ala Gly Gln Tyr Gln Gln Gly
                   405                 410                 415
   Pro Gly Gln Gln Gly Pro Tyr Gly Pro Gly Ala Ser Gly Pro Gly Gln
               420                 425                 430
   Gln Gly Pro Tyr Gly Pro Gly Ala Ser Ala Ala Ala Ala Ala Gly Pro
           435                 440                 445
   Gly Gln Tyr Val Leu Ile Gly Pro Gly Gln Gln Val Leu Ile Gly Pro
       450                 455                 460
   Ser Ala Ser Ala Ala Ala Ala Ala Gly Gln Tyr Gly Ser Gly Pro Gly
   465                 470                 475                 480
   Gln Tyr Gly Pro Tyr Gly Pro Gly Gln Ser Gly Pro Gly Ser Gly Gln
                   485                 490                 495
   Gln Gly Gln Gly Pro Tyr Gly Pro Gly Ala Ser Ala Ala Ala Ala Ala
               500                 505                 510
   Gly Ser Tyr Gly Pro Gly Gln Gln Val Leu Ile Gly Pro Tyr Val Leu
           515                 520                 525
   Ile Gly Pro Gly Pro Ser Ala Ala Ala Ala Ala Gly Pro Gly Ser Gly
       530                 535                 540
   Gln Tyr Gly Pro Gly Ala Ser Gly Gln Asn Gly Pro Gly Ser Gly Gln
   545                 550                 555                 560
   Tyr Gly Pro Gly Gln Gln Gly Pro Gly Gln Ser Ala Ala Ala Ala Ala
                   565                 570                 575
   Gly Gln Tyr Gln Gln Val Leu Ile Gly Pro Gly Gln Gln Gly Pro Tyr
               580                 585                 590
   Val Leu Ile Gly Pro Gly Ala Ser Ala Ala Ala Ala Ala Gly Pro Gly
           595                 600                 605
   Ser Gly Gln Gln Val Leu Ile Gly Pro Gly Ala Ser Val Leu Ile
       610                 615                 620

<210>  9
<211>  576
<212>  PRT
<213>  Artificial Sequence

<220>
<223>  GEN514

<400>  9

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Gly Pro Gly
65                  70                  75                  80

Gln Gln Gly Pro Ser Ala Ser Ala Ala Ala Ala Ala Ala Ala Gly Pro
                85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala
        115                 120                 125

Ala Ala Ala Gly Ser Tyr Gly Ser Gly Pro Gly Gln Gln Gly Pro Tyr
    130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Gln Gln Gly Pro Ser Ala Ser Ala Ala Ala Ala Ala Ala Ala
            180                 185                 190

Gly Ser Gly Gln Gln Gly Pro Gly Gln Tyr Gly Pro Tyr Ala Ser Ala
        195                 200                 205

Ala Ala Ala Ala Ala Ala Gly Ser Tyr Gly Ser Gly Pro Gly Gln Gln
    210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Ala Gly Pro
                245                 250                 255

Gly Gln Gln Gly Pro Tyr Gly Pro Gly Ser Ser Ala Ala Ala Ala Ala
            260                 265                 270

Ala Ala Gly Ser Tyr Gly Tyr Gly Pro Gly Gln Gln Gly Pro Tyr Gly
        275                 280                 285

Pro Gly Ala Ser Gly Gln Asn Gly Pro Gly Ser Gly Gln Tyr Gly Pro
    290                 295                 300

Gly Gln Gln Gly Pro Gly Pro Ser Ala Ala Ala Ala Ala Ala Ala Gly
305                 310                 315                 320

Pro Gly Gln Gln Gly Pro Tyr Gly Pro Gly Ala Ser Ala Ala Ala Ala
                325                 330                 335

Ala Ala Ala Gly Ser Tyr Gly Pro Gly Gln Gln Gly Pro Gly Gln Tyr
            340                 345                 350

Gly Pro Gly Ser Ser Gly Pro Gly Gln Gln Gly Pro Tyr Gly Pro Gly
        355                 360                 365

Ser Ser Ala Ala Ala Ala Ala Ala Ala Gly Ser Tyr Gly Pro Gly Gln
    370                 375                 380

Gln Gly Pro Tyr Gly Pro Gly Pro Ser Ala Ala Ala Ala Ala Ala Ala
385                 390                 395                 400

Gly Ser Tyr Gln Gln Gly Pro Gly Gln Gln Gly Pro Tyr Gly Pro Gly
                405                 410                 415

Ala Ser Gly Pro Gly Gln Gln Gly Pro Tyr Gly Pro Gly Ala Ser Ala
            420                 425                 430

Ala Ala Ala Ala Ala Ala Gly Pro Gly Gln Tyr Gly Pro Gly Gln Gln
        435                 440                 445

Gly Pro Ser Ala Ser Ala Ala Ala Ala Ala Ala Ala Gly Ser Tyr Gly
    450                 455                 460

Ser Gly Pro Gly Gln Tyr Gly Pro Tyr Gly Pro Gly Gln Ser Gly Pro
465                 470                 475                 480

Gly Ser Gly Gln Gln Gly Gln Gly Pro Tyr Gly Pro Gly Ala Ser Ala
                485                 490                 495

Ala Ala Ala Ala Ala Ala Gly Ser Tyr Gly Pro Gly Gln Gln Gly Pro
            500                 505                 510

Tyr Gly Pro Gly Pro Ser Ala Ala Ala Ala Ala Ala Ala Gly Pro Gly
        515                 520                 525

Ser Gly Gln Tyr Gly Pro Gly Ala Ser Gly Gln Asn Gly Pro Gly Ser
    530                 535                 540

Gly Gln Tyr Gly Pro Gly Gln Gln Gly Pro Gly Pro Ser Ala Ala Ala
545                 550                 555                 560

Ala Ala Ala Ala Gly Pro Gly Ser Gly Gln Gln Gly Pro Gly Ala Ser
                565                 570                 575

<210>  10
<211>  603
<212>  PRT
<213>  Artificial Sequence

<220>
<223>  GEN767

<400>  10

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Val Leu Ile
65                  70                  75                  80

Gly Pro Gly Gln Gln Gly Pro Ser Ala Ser Ala Ala Ala Ala Ala Ala
                85                  90                  95

Ala Gly Pro Gly Ser Gly Gln Gln Gly Pro Gly Ala Ser Gly Gln Tyr
            100                 105                 110

Gly Pro Gly Gln Gln Gly Pro Gly Gln Gln Gly Pro Gly Ser Ser Ala
        115                 120                 125

Ala Ala Ala Ala Ala Ala Gly Ser Tyr Gly Ser Val Leu Ile Gly Pro
    130                 135                 140

Gly Gln Gln Gly Pro Tyr Gly Ser Ala Ala Ala Ala Ala Ala Ala Gly
145                 150                 155                 160

Pro Gly Ser Gly Gln Tyr Gly Gln Gly Pro Tyr Gly Pro Gly Ala Ser
                165                 170                 175

Gly Pro Gly Gln Tyr Gly Pro Gly Gln Gln Gly Pro Ser Ala Ser Ala
            180                 185                 190

Ala Ala Ala Ala Ala Ala Gly Ser Gly Gln Gln Val Leu Ile Gly Pro
        195                 200                 205

Gly Gln Tyr Gly Pro Tyr Ala Ser Ala Ala Ala Ala Ala Ala Ala Gly
    210                 215                 220

Ser Tyr Gly Ser Gly Pro Gly Gln Gln Gly Pro Tyr Gly Pro Gly Gln
225                 230                 235                 240

Ser Gly Ser Gly Gln Gln Gly Pro Gly Gln Gln Gly Pro Tyr Ala Ser
                245                 250                 255

Ala Ala Ala Ala Ala Ala Ala Gly Pro Gly Gln Gln Val Leu Ile Gly
            260                 265                 270

Pro Tyr Gly Pro Gly Ser Ser Ala Ala Ala Ala Ala Ala Ala Gly Ser
        275                 280                 285

Tyr Gly Tyr Gly Pro Gly Gln Gln Gly Pro Tyr Gly Pro Gly Ala Ser
    290                 295                 300

Gly Gln Asn Gly Pro Gly Ser Gly Gln Tyr Gly Pro Gly Gln Gln Gly
305                 310                 315                 320

Pro Gly Pro Ser Ala Ala Ala Ala Ala Ala Ala Gly Pro Gly Gln Gln
                325                 330                 335

Val Leu Ile Gly Pro Tyr Gly Pro Gly Ala Ser Ala Ala Ala Ala Ala
            340                 345                 350

Ala Ala Gly Ser Tyr Gly Pro Gly Gln Gln Gly Pro Gly Gln Tyr Gly
        355                 360                 365

Pro Gly Ser Ser Gly Pro Gly Gln Gln Gly Pro Tyr Gly Pro Gly Ser
    370                 375                 380

Ser Ala Ala Ala Ala Ala Ala Ala Gly Ser Tyr Gly Pro Gly Gln Gln
385                 390                 395                 400

Val Leu Ile Gly Pro Tyr Gly Pro Gly Pro Ser Ala Ala Ala Ala Ala
                405                 410                 415

Ala Ala Gly Ser Tyr Gln Gln Gly Pro Gly Gln Gln Gly Pro Tyr Gly
            420                 425                 430

Pro Gly Ala Ser Gly Pro Gly Gln Gln Gly Pro Tyr Gly Pro Gly Ala
        435                 440                 445

Ser Ala Ala Ala Ala Ala Ala Ala Gly Pro Gly Gln Tyr Val Leu Ile
    450                 455                 460

Gly Pro Gly Gln Gln Gly Pro Ser Ala Ser Ala Ala Ala Ala Ala Ala
465                 470                 475                 480

Ala Gly Ser Tyr Gly Ser Gly Pro Gly Gln Tyr Gly Pro Tyr Gly Pro
                485                 490                 495

Gly Gln Ser Gly Pro Gly Ser Gly Gln Gln Gly Gln Gly Pro Tyr Gly

500                 505                 510

Pro Gly Ala Ser Ala Ala Ala Ala Ala Ala Ala Gly Ser Tyr Gly Pro
        515                 520                 525

Gly Gln Gln Val Leu Ile Gly Pro Tyr Gly Pro Gly Pro Ser Ala Ala
    530                 535                 540

Ala Ala Ala Ala Ala Gly Pro Gly Ser Gly Gln Tyr Gly Pro Gly Ala
545                 550                 555                 560

Ser Gly Gln Asn Gly Pro Gly Ser Gly Gln Tyr Gly Pro Gly Gln Gln
                565                 570                 575

Gly Pro Gly Pro Ser Ala Ala Ala Ala Ala Ala Ala Gly Pro Gly Ser
            580                 585                 590

Gly Gln Gln Gly Pro Gly Ala Ser Val Leu Ile
        595                 600

<210>  11
<211>  630
<212>  PRT
<213>  Artificial Sequence

<220>
<223>  GEN765

<400>  11

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Val Leu Ile
65                  70                  75                  80

Gly Pro Gly Gln Gln Val Leu Ile Gly Pro Ser Ala Ser Ala Ala Ala
                85                  90                  95

Ala Ala Ala Ala Gly Pro Gly Ser Gly Gln Gln Gly Pro Gly Ala Ser
            100                 105                 110

Gly Gln Tyr Gly Pro Gly Gln Gln Gly Pro Gly Gln Gln Gly Pro Gly
        115                 120                 125

Ser Ser Ala Ala Ala Ala Ala Ala Ala Gly Ser Tyr Gly Ser Val Leu
    130                 135                 140

Ile Gly Pro Gly Gln Gln Val Leu Ile Gly Pro Tyr Gly Ser Ala Ala
145                 150                 155                 160

Ala Ala Ala Ala Ala Gly Pro Gly Ser Gly Gln Tyr Gly Gln Gly Pro
                165                 170                 175

Tyr Gly Pro Gly Ala Ser Gly Pro Gly Gln Tyr Gly Pro Gly Gln Gln
            180                 185                 190

Gly Pro Ser Ala Ser Ala Ala Ala Ala Ala Ala Ala Gly Ser Gly Gln
        195                 200                 205

Gln Val Leu Ile Gly Pro Gly Gln Tyr Val Leu Ile Gly Pro Tyr Ala
    210                 215                 220

Ser Ala Ala Ala Ala Ala Ala Ala Gly Ser Tyr Gly Ser Gly Pro Gly
225                 230                 235                 240

Gln Gln Gly Pro Tyr Gly Pro Gly Gln Ser Gly Ser Gly Gln Gln Gly
                245                 250                 255

Pro Gly Gln Gln Gly Pro Tyr Ala Ser Ala Ala Ala Ala Ala Ala Ala
            260                 265                 270

Gly Pro Gly Gln Gln Val Leu Ile Gly Pro Tyr Val Leu Ile Gly Pro
        275                 280                 285

Gly Ser Ser Ala Ala Ala Ala Ala Ala Ala Gly Ser Tyr Gly Tyr Gly
    290                 295                 300

Pro Gly Gln Gln Gly Pro Tyr Gly Pro Gly Ala Ser Gly Gln Asn Gly
305                 310                 315                 320

Pro Gly Ser Gly Gln Tyr Gly Pro Gly Gln Gln Gly Pro Gly Pro Ser
                325                 330                 335

Ala Ala Ala Ala Ala Ala Ala Gly Pro Gly Gln Gln Val Leu Ile Gly
            340                 345                 350

Pro Tyr Val Leu Ile Gly Pro Gly Ala Ser Ala Ala Ala Ala Ala Ala

355                 360                 365

Ala Gly Ser Tyr Gly Pro Gly Gln Gln Gly Pro Gly Gln Tyr Gly Pro
    370                 375                 380

Gly Ser Ser Gly Pro Gly Gln Gln Gly Pro Tyr Gly Pro Gly Ser Ser
385                 390                 395                 400

Ala Ala Ala Ala Ala Ala Ala Gly Ser Tyr Gly Pro Gly Gln Gln Val
                405                 410                 415

Leu Ile Gly Pro Tyr Val Leu Ile Gly Pro Gly Pro Ser Ala Ala Ala
            420                 425                 430

Ala Ala Ala Ala Gly Ser Tyr Gln Gln Gly Pro Gly Gln Gln Gly Pro
        435                 440                 445

Tyr Gly Pro Gly Ala Ser Gly Pro Gly Gln Gln Gly Pro Tyr Gly Pro
    450                 455                 460

Gly Ala Ser Ala Ala Ala Ala Ala Ala Ala Gly Pro Gly Gln Tyr Val
465                 470                 475                 480

Leu Ile Gly Pro Gly Gln Gln Val Leu Ile Gly Pro Ser Ala Ser Ala
                485                 490                 495

Ala Ala Ala Ala Ala Ala Gly Ser Tyr Gly Ser Gly Pro Gly Gln Tyr
            500                 505                 510

Gly Pro Tyr Gly Pro Gly Gln Ser Gly Pro Gly Ser Gly Gln Gln Gly
        515                 520                 525

Gln Gly Pro Tyr Gly Pro Gly Ala Ser Ala Ala Ala Ala Ala Ala Ala
    530                 535                 540

Gly Ser Tyr Gly Pro Gly Gln Gln Val Leu Ile Gly Pro Tyr Val Leu
545                 550                 555                 560

Ile Gly Pro Gly Pro Ser Ala Ala Ala Ala Ala Ala Ala Gly Pro Gly
                565                 570                 575

Ser Gly Gln Tyr Gly Pro Gly Ala Ser Gly Gln Asn Gly Pro Gly Ser
            580                 585                 590

Gly Gln Tyr Gly Pro Gly Gln Gln Gly Pro Gly Pro Ser Ala Ala Ala
        595                 600                 605

Ala Ala Ala Ala Gly Pro Gly Ser Gly Gln Gln Val Leu Ile Gly Pro
    610                 615                 620

Gly Ala Ser Val Leu Ile
625                 630